# EUROPEAN PATENT APPLICATION

(11) **EP 3 088 076 A1**
(43) Date of publication of application: **02.11.2016**
(21) Application number: 14874696.9
(22) Date of filing: 19.12.2014
(51) Int. Cl.: B01J 19/00, C12M 1/00, G01N 37/00

(54) **THREE-DIMENSIONAL MICROCHEMICAL CHIP**

(30) Priority: 27.12.2013 JP 2013272327
(71) Applicant: ASAHI FR R&D Co., Ltd., Saitama-shi, Saitama 330-0801 (JP)
(72) Inventor: TAKAGI, Kazuhisa, Saitama-shi Saitama 330-0801 (JP); TAKANO, Tsutomu, Saitama-shi Saitama 330-0801 (JP); UBUKATA, Yuya, Saitama-shi Saitama 330-0801 (JP)
(74) Representative: dompatent von Kreisler Selting Werner - Partnerschaft von Patent- und Rechtsanwälten mbB
(86) International application number: PCT/JP2014/083649
(87) International publication number: WO 2015/098720

(57) **Abstract**

Provided is a simple, compact and steric three-dimensional microchemical chip, that can be produced with a high yield on a large scale and so as to be homogeneous quality; in which a long micro flow path, which is made to flow a fluid sample by pressurization or by capillarity phenomenon, is sterically and reliably defined so that the fluid sample can be precisely and reliably flowed into the flow path as desired and can accurately, simply, quickly and precisely be subjected to chemical reaction and chemical action as desired.

A three-dimensional microchemical chip 1 comprises flow-path-supporting substrate sheets 20, 40, flow-path-retaining substrate sheets 10, 30, 50 which is stacked to the flow-path-supporting substrate sheets 20, 40 and join and integrate therewith by a direct covalent bond or an indirect covalent bond via molecular adhesive, flow paths 22, 42 defined by recessing and/or piercing the flow-path-supporting substrate sheets 20, 40 and sterically and sequentially, in which a fluid sample is subjected to a chemical reaction and/or chemical action, a receiving hole which is pierced in the flow-path-retaining substrate sheet 30 and is connected to the flow paths 22, 42; the flow paths 22, 42 are sequentially and sterically connected from fluid-sample-injecting holes 11 to fluid-sample-draining holes13.

## Description

### Field of Invention

The present invention relates to a sterical and compact three-dimensional microchemical chip which is used by being installed to an microanalysis apparatus for conducting microanalysis by flowing a biological component, which is included into a test sample of a specimen originated from a biological object, into a micro flow path, a microreactor for chemical microsynthesis of a useful substance by flowing a raw material component of a useful substance such as the biological component etc. exhibiting pharmacological action and a reagent of a reactive substrate etc. into a micro flow path, and a micro-biochemical treatment apparatus for multiplication of cells etc.

### Background of the Invention and Related Art Statement

In order to quantitate a reaction amount of an enzyme which acts on a substrate in a specimen or an amount of the substrate by degree of color generation depending on a reagent which is colored by the enzyme or substrate, a microbiological chip has been used. In this regard, specific substrate selectivity of the enzyme is utilized by using a trace amount like µL order of a test sample such as blood, urine and the like, which are the specimen originated from a biological object. Further, when a quantitative analysis of the substrate amount by converting the enzyme reaction amount into an electric signal by using a membrane including the enzyme and electrodes, DNA extraction and a polymerase chain reaction (PCR) amplification thereof, or ion concentration measurement, microsynthesis etc. of nucleic acid, saccharide, protein or peptide is conducted in µM order, a microreactor chip has been used. Furthermore a micro-biochemical chip has been used to multiply useful cells and virus, and to attach cancer cells for an examination.

A microchemical chip such as the microbiological chip, microreactor chip and micro-biochemical chip has a channel-shaped micro flow path as a reaction channel which mixes, reacts, separates, attaches and detects a fluid sample exemplified with a specimen such as suspension and a solution, a reagent of liquid and a sample such as cell sap which are pressurized, imported and flowed thereinto.

In the microchemical chip, the micro flow path of several dozen to several hundreds micrometers is defined on an inorganic substrate such as a stainless substrate, silicon substrate, quartz substrate and glass substrate, and an organic substrate of a resin substrate or a rubber substrate by means of cutting or etching.

For instance, Patent Document I discloses that a microchemical chip having the micro flow path, which is made of the organic substrate, is formed by employing a high-transparent plastic resin. Because the resin substrate and a rubber substrate are easy to be shaped or cut, the microchemical chip, which is formed by gluing these substrates using adhesive agent or by heat sealing, is suitable for producing on the large scale. Especially, the microchemical chip made of the transparent organic substrate or transparent inorganic substrate is useful to an optical system analysis.

Because the flat organic substrate is difficult to deteriorate against a water soluble specimen and/or reagents such as strong acids of hydrofluoric acid etc. which solve a metal, or a water soluble chemical agent, the microchemical chip made of the organic substrate is chemically and biochemically stable.

Because the microchemical chip is installed to the microanalysis apparatus and the microreactor in existence, size thereof cannot get so large. Accordingly the micro flow path in the microchemical chip has been needed to be defined on a limited plain face thereof. In addition because a resin sheet or rubber sheet having the flow path is adhered through the adhesive agent or sealed by heating, the microchemical chip has low bonding strength. When the high-pressurized specimen, reagent and/or sample flow into the flow path, the bonded substrates cannot withstand the pressure and break up adhesion therebetween and thus, the microchemical chip is physically weak and is easily broken. Even if the specimen, reagent and/or sample is pressurized by slightly high pressure so as to avoid breakdown of the microchemical chip in order to flow the specimen, reagent or sample into the fine, branched, and complex-patterned flow path, the specimen, reagent or sample has been difficult to reach the terminal thereof. Because interposition of the adhesive agent or overheating has caused outflow of the adhesive agent to the flow path, fluctuation of a refractive index or heat deforming and distortion, the microchemical chip made of the transparent resin sheets having the flow path bonded by adhering through the adhesive agent or by heat sealing has been difficult to have homogeneous transparency, which is important to the fine optical system analysis or observance, on the flow path.

### [Prior Art Document]

### [Patent Document]

[Patent Document 1] Japanese Patent Application Publication No. 2006-218611

### Problems to Be Solved by the Invention

The present invention was made in view of solving the above described problems, and its object is to provide a simple, compact and steric three-dimensional microchemical chip, that can be produced with a high yield on a large scale and so as to be homogeneous quality, in which a long micro flow path, which is made to flow a valuable specimen and sample such as a slightly specimen originated from a biological object and/or a dilute reagent of a trace amount by high pressurization or by capillarity phenomenon as desired, is sterically and reliably defined so that these fluid samples can be accurately and reliably imported into the flow path as desired by the long and three-dimensionally defined micro flow path, an useful substance such as a biological component, reagent and cells included in the specimen is accurately, simply, quickly and precisely analyzed and reacted and can be chemically reacted accordingly, or is attached, adsorbed or multiplied by reaching it and can be chemically or biochemically acted.

### Brief Summary of the Invention

A three-dimensional microchemical chip developed to achieve the objects above described comprises: a single or plural flow-path-supporting substrate sheet made from rubber, resin, metal, ceramics and/or glass; a flow-path-retaining substrate sheet made from rubber, resin, metal, ceramics and/or glass, and retains to contact and stack the flow-path-supporting substrate sheet at most upper face and/or most lower face thereof; a surface of at least one of the flow-path-supporting substrate sheet and the flow-path-retaining substrate sheet which joins and integrates these sheets by a direct covalent bond and/or indirect covalent bond interposing a molecular adhesive via a treatment at least one of a dry treatment selected from the group consisting of a corona treatment, plasma treatment and ultraviolet irradiation treatment, and a molecular adhesive treatment; a flow path, defined by recessing and/or piercing the flow-path-supporting substrate sheet, in which a fluid sample, selected from the group consisting of a specimen, reagent and sample, is subjected to a chemical reaction and/or chemical action by flowing the fluid sample thereinto through pressurization and/or capillarity phenomenon thereof; and a receiving hole which is pierced in the flow-path-retaining substrate sheet covering the flow path and is connected to the flow path, the flow path and the receiving hole are sequentially and sterically connected from a fluid-sample-injecting hole to a fluid-sample-draining hole. As long as irradiation of ultraviolet is conducted, the ultraviolet irradiation treatment is not restricted and may be a usual ultraviolet irradiation treatment (an UV treatment) which is irradiation of a broadband wavelength or multiple wavelengths and may be an excimer ultraviolet treatment (an excimer UV treatment) which is irradiation of excimer ultraviolet. The excimer ultraviolet may be seen as a single wavelength.

In the three-dimensional microchemical chip, at least any one of the flow path of the plural flow-path-supporting substrate sheets may be folded, bent and/or curved at least one location of a midway part thereof.

In the three-dimensional microchemical chip, diatomaceous earth, mica, talc and/or kaolin is preferably included in any one of the flow-path-supporting substrate sheet and the flow-path-retaining substrate sheet.

In the three-dimensional microchemical chip, the flow-path-supporting substrate sheet and the flow-path-retaining substrate sheet may be plurally and alternately stacked each other.

In the three-dimensional microchemical chip, any one of the flow path of the plural flow-path-supporting substrate sheets is preferably folded back from a draining side toward an injecting side at least one location of a midway part thereof.

In the three-dimensional microchemical chip, the flow path on the plural flow-path-supporting substrate sheets may be parallel arranged, diagonally crossed and/or not diagonally crossed in different level in at least one part thereof each other.

In the three-dimensional microchemical chip, any one of the flow-path-supporting substrate sheet and the flow-path-retaining substrate sheet may be silicone rubber-made thermal radiation sheet including a thermally conductive filler powder at least one selected from the group consisting of aluminum oxide, magnesium oxide, zinc oxide, graphite carbon, silicon nitride, boron nitride and aluminum nitride.

In the three-dimensional microchemical chip, the molecular adhesive is preferably included in any one of the flow-path-supporting substrate sheet and the flow-path-retaining substrate sheet.

In the three-dimensional microchemical chip, the flow-path-supporting substrate sheet and the flow-path-retaining substrate sheet may be joined through the molecular adhesive on the surface of these sheets.

In the three-dimensional microchemical chip, the molecular adhesive may contain a silane coupling agent having 6 to 12 carbon atoms and a vinylmethoxysilyl group.

In the three-dimensional microchemical chip, a flame retardant of at least one selected from the group consisting of antimony trioxide and aluminum hydroxide is preferably contained in any one of the flow-path-supporting substrate sheet and the flow-path-retaining substrate sheet.

In the three-dimensional microchemical chip, the covalent bond may be an ether bond.

In the three-dimensional microchemical chip, at least one part of the flow-path-supporting substrate sheet or the flow-path-retaining substrate sheet may a foamable silicone rubber sheet made from at least any one from the group consisting of a silicone rubber raw material composite including glass beads and/or zeolite and a silicone composite including a water soluble alcohol.

In the three-dimensional microchemical chip, any one of the flow-path-supporting substrate sheet and the flow-path-retaining substrate sheet may be a high-reflective silicone rubber sheet, having 80 to 100% of reflectivity, made from silicone rubber into which anatase-type or rutile-type titanium oxide is dispersed.

In the three-dimensional microchemical chip, any one of the flow-path-supporting substrate sheet and the flow-path-retaining substrate sheet may be a low-gas-permeable silicone rubber sheet, having 500 to 0.05 (cc/cm²/mm/sec/cm·Hg×10¹⁰) of gas permeability of at least any gas of oxide gas, nitrogen gas, carbon dioxide gas and water vapor, which is made from silicone rubber, ethylene-propylene-diene-methylene copolymer rubber, butyl rubber, acrylonitrile-butadiene copolymer rubber, fluoro-rubber, styrene-butadiene copolymer rubber and/or hydrin rubber.

In the three-dimensional microchemical chip, at least any one of the diatomaceous earth, the mica, the talc and the kaolin may be scaly-shaped filler.

In the three-dimensional microchemical chip, at least any one of the flow-path-supporting substrate sheet and the flow-path-retaining substrate sheet may be formed by any one selected from the group consisting of peroxide crosslinking silicone rubber, addition crosslinking silicone rubber, condensation crosslinking silicone rubber and radiation crosslinking or electron beam crosslinking silicone rubber, and blended rubber of any one the silicone rubbers and olefin type rubber.

In the three-dimensional microchemical chip, a part of the flow path may be a fluid accumulation part defined by expansion thereof.

A method for producing a three-dimensional microchemical chip comprises a flow path defining step for defining a flow path for a chemical reaction and/or chemical action, into which a fluid sample selected from the group consisting of a specimen, reagent and sample is flowed by pressurization and/or capillarity phenomenon, in a single or plural flow-path-supporting substrate sheet so as to sequentially and sterically connect from a fluid-sample-injecting hole to a fluid-sample-draining hole; a receiving hole forming step for producing a flow-path-retaining substrate sheet which is made from rubber, resin, metal, ceramics and/or glass, has a receiving hole connecting to the flow path, and sandwiches the flow-path-supporting substrate sheet; a treating step for conducting a corona treatment, plasma treatment or ultraviolet irradiation treatment to at least any one of the flow-path-supporting substrate sheet and the flow-path-retaining substrate sheet; a joining step for stacking the flow-path-supporting substrate sheet and the flow-path-retaining substrate sheet under conditions of normal atmospheric pressure, pressurization or reduced pressure, joining and integrating these sheets via a direct covalent bond and/or indirect covalent bond interposing a molecular adhesive, whereby each of the flow path of the flow-path-supporting substrate sheet is sequentially and sterically connected from the fluid-sample-injecting hole the fluid-sample-draining hole.

In the method for producing the three-dimensional microchemical chip, a step for forming the flow-path-supporting substrate sheet so that at least one location in at least any one of the flow path of the flow-path-supporting substrate sheet is preferably folded, bent and/or curved halfway.

### Effects of the Invention

In the three-dimensional microchemical chip of the present invention, the long and micro flow path, which makes a valuable specimen such as a slightly specimen originated from a biological object, and a dilute reagent and cells of a trace amount by pressurization using high pressure or by capillarity phenomenon flow, is sterically and reliably defined.

According to the three-dimensional microchemical chip, when the micro flow path is folded, bent and/or curved on at least one location of the midway part thereof while connecting from the fluid-sample-injecting hole to the fluid-sample-draining hole, the micro flow path can be sequentially and sterically defined. Thereby the micro flow path can be allowed for a further distance. Because the three-dimensional microchemical chip has the long and sterical micro flow path, a chemical reaction can be progressed enough at the micro flow path halfway. In the result, the biological component in the specimen and the useful substance of the reagent are accurately, simply, quickly and precisely analyzed and reacted, or the sample of the cells can be prepared.

In the three-dimensional microchemical chip, the micro flow path may be folded, bent and/or curved on at least one location of a midway part thereof, defined per the plural flow-path-supporting substrate sheets, and parallel arranged, diagonally crossed and/or not diagonally crossed in different level. Thus the three-dimensional microchemical chip achieves both of the long micro flow path and the compact size thereof. The micro flow path is preferably folded back from the draining side toward the injecting side to prevent overpressure when fluid flowing.

When the three-dimensional microchemical chip has the silicone rubber-made thermal radiation sheet including the thermally conductive filler powder, heat radiation easily occurs. Thus when the fluid sample is flowed into the fine flow path by the high pressurization and/or the capillarity phenomenon, cooling thereof occurs sufficiently. The fluid sample such as the valuable specimen as a slightly specimen originated from a biological object and/or the diluted reagent of a trace amount is not exposed to high temperature and thus, unexpected decomposition thereof and conversion into impurity via a side reaction do not occur. Analyzing and reacting of the fluid sample therefore can be conducted as desired. Further, the three-dimensional microchemical chip preferably has the silicone rubber-made thermally conductive sheet. Because the silicone rubber-made thermally conductive sheet easily conducts heat, the three-dimensional microchemical chip may be heated rapidly, and can be maintained at optimum temperatures while heating by a heater intentionally.

In the three-dimensional microchemical chip, the flow-path-supporting substrate sheet and the flow-path-retaining substrate sheet are tightly joined at bonded faces except flow path regions on these sheets by strong adhesive. The strong adhesive may be obtained by a direct-chemical-intermolecularly bond e.g. the covalent bond such as the ether bond. Hence the fine flow path, which can be flowed the fluid sample by high pressurization without leakage thereof, is reliably defined.

In the three-dimensional microchemical chip, the fine flow path from 0.5 µm to 5 mm in width having a linear shape combined with a straight line and curved line or complex pattern shape, which is expanded, focused or branched at the terminal or midway part thereof, is accurately defined in each of the plural flow-path-supporting substrate sheets. In spite of having such the fine flow path, when the fluid sample of the specimen, the reagent and/or the sample is imported into the flow path by pressurization, and is flowed therein, the flow-path-supporting substrate sheets and the flow-path-retaining substrate sheets do not break up adhesions therebetween. Thus, the three-dimensional microchemical chip does not break.

In the three-dimensional microchemical chip, even if the fluid sample of the liquid or gaseous specimen, reagent and/or sample is imported into the fine flow path by pressurization from normal atmospheric pressure to about 5 atmospheric pressures, the flow path does not break due to elasticity of the flow-path-supporting substrate sheet. Even if the fluid sample as above is imported into the fine flow path at a low or high temperature range from about freezing temperature to 120°C, generally at 0 to 100°C or at 20 to 120°C while repeating heating and cooling, the flow path does not break, similarly.

According to the three-dimensional microchemical chip, the fluid sample of the specimen, reagent and/or sample can be reliably and accurately imported into the desired flow path while maintaining at a specific temperature. In the result, analyzing of an useful substance such as a biological component etc. in the specimen can be conducted on a short period precisely and simply, the reaction of a raw material component of the useful substance such as the biological component etc. exhibiting pharmacological action or a reagent of reactive substrate etc. can be progressed so as to serve purposes, and the reach of desired cells can be achieved so as to serve purposes.

The three-dimensional microchemical chip inhibits contact between the specimen, reagent and/or sample and the rubber sheets as far as possible due to the fine flow path of the rubber sheet, and can prevent contamination and adsorption of the specimen, reagent and/or sample.

When the three-dimensional microchemical chip is used and thrown away, pollution could not occur by contamination of another specimen, reagent and/or sample and thus, reliable results can be obtained.

Because the three-dimensional microchemical chip has the micro flow path which is sterically defined, the micro flow path having long distance, an external shape of square of several millimeters to several dozen of centimeters having an extremely compact size, and a simple component are simultaneously achieved. The three-dimensional microchemical chip includes the numerous and serial, parallel or branched flow path planarly and vertically in spite of the compact size, and may have injecting holes and draining holes. Thus, the three-dimensional microchemical chip may give numerous functions so that plural reactions are progressed through many processes in series, parallel or steric and crossed. By using a portable analytical apparatus without a large-scaled analysis apparatus, a plurality of qualitative or quantitative analyses therefore can be swiftly conducted at not only inside but also outside. Further, an amount of an analytical reagent and a reactive reagent used in the three-dimensional microchemical chip can be restrained to be a small amount. In addition, since an amount of a waste fluid is much more less than an analysis or reaction by using a flask or test tube, the three-dimensional microchemical chip helps environmental protection.

The method for producing of the three-dimensional microchemical chip of the present invention is extremely simple and has short processes, and the three-dimensional microchemical chip can be produced with high quality, homogeneity, low cost and high yield on the large scale.

According to the method therefor, the flow path, which flows the fluid sample such as the specimen, reagent and sample thereinto by the high pressurization and/or the capillarity phenomenon and conducts various chemical reactions/chemical actions, is defined in the single or plural flow-path-supporting substrate sheet made from rubber. The micro flow path can be defined so that each of the flow paths sterically and sequentially connects from the fluid-sample-injecting holes to the fluid-sample-draining holes, and is folded back opposite from the draining side on at least one location of a midway part thereof in the plural flow-path-supporting substrate sheets. The micro flow path can be easily produced.

Further, according to the method therefor, the covalent bond such as the ether bond is directly formed between the flow-path-supporting substrate sheet and the flow-path-retaining substrate sheet except flow path regions of these sheets by contacting thereof. The covalent bond is easily formed and has considerably high joining strength as compared to adhesive agents. The molecular adhesion can be generated enough under conditions of normal atmospheric pressure, pressurization or reduced pressure by optionally heating at a temperature less than a thermal-welding temperature of thermoplastic resin for a short time. Thereby high temperature heating comparable to the thermal-welding temperature thereof is not needed, and variation of a reflective index and thermal deformation/distortion, which decrease accuracy of optical analysis, do not occur.

### Brief Description of Several Views of Drawings

Fig. 1 is a schematic exploded perspective view showing an embodiment of the three-dimensional microchemical chip of the present invention.
Fig. 2 is a partially broken away schematic perspective view showing an embodiment of the three-dimensional microchemical chip of the present invention.
Fig. 3 is a perspective view showing an embodiment of a state in which the three-dimensional microchemical chip of the present invention is used.
Fig. 4 is a partially broken away schematic perspective view showing another embodiment of the three-dimensional microchemical chip of the present invention.
Fig. 5 is a schematic exploded perspective view showing the other embodiment of the three-dimensional microchemical chip of the present invention.
Fig. 6 is a schematic exploded perspective view showing the other embodiment of the three-dimensional microchemical chip of the present invention.
Fig.7 is a graph showing relations between a temperature and period of time in regard to thermal insulation properties of the silicone rubber sheet which may be employed in the flow-path-supporting substrate sheet of the three-dimensional microchemical chip of the present invention.

### Detailed Description of the Invention

Hereunder, preferred embodiments to practice the present invention in detail will be explained, but the scope of the present invention is not restricted by these embodiments.

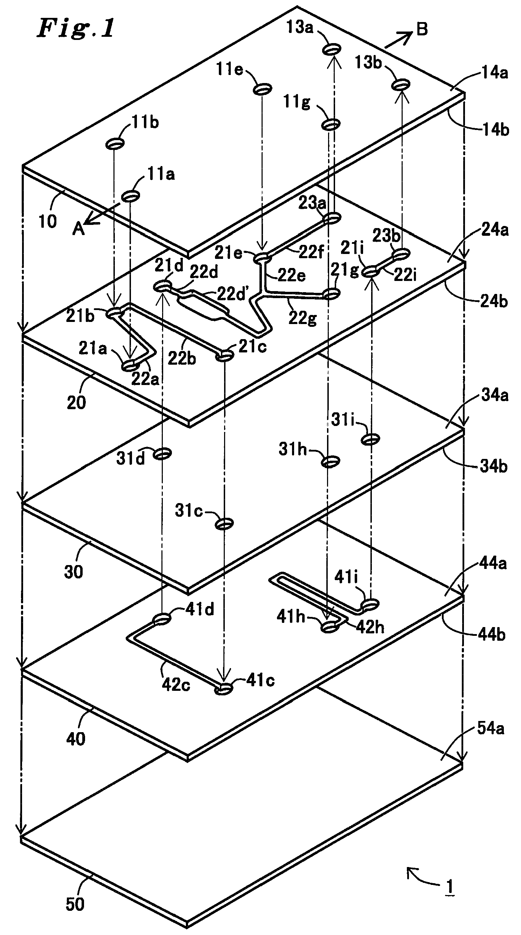

An embodiment of a three-dimensional microchemical chip 1 of the present invention is shown in Fig. 1 of a schematic exploded perspective view illustrating a procedure of producing the three-dimensional microchemical chip 1. The three-dimensional microchemical chip 1 is prepared by stacking silicone rubber-made flow-path-supporting substrate sheets 20, 40 having a fine flow path and a polyimide-made flow-path-retaining substrate sheet 30 having piercing holes 31 connecting the flow paths of each sheet 10 to 50 between a polyimide-made flow-path-retaining substrate sheet 50 for supporting of the bottom and a polyimide-made flow-path-retaining substrate sheet 10 for covering. The three-dimensional microchemical chip 1 has flexibility.

Fluid-sample-injecting holes 11a, 11b, 11e, 11g and fluid-sample-draining holes 13a, 13b are pierced in the both faces of the flow-path-retaining substrate sheet 10, and exposed on a first face 14a.

The flow-path-supporting substrate sheet 20 has a fluid-sample-receiving hole 21 a corresponding to the fluid-sample-injecting hole 11a, a micro flow path 22a extending therefrom, a fluid-sample-receiving hole 21b at a bent part thereof, a micro flow path 22b extending therefrom and bending halfway and a fluid-sample-receiving hole 21c at a terminal part thereof while piercing the both faces thereof. In addition, the flow-path-supporting substrate sheet 20 has a micro flow path 22d which is extended from a fluid-sample-receiving hole 21 d and is bent halfway, a fluid accumulation part 22d' expanded at a midway part of the micro flow path 22d, micro flow paths 22e, 22g branching at a downstream side thereof, a fluid-sample-receiving hole 21e corresponding to the fluid-sample-injecting hole 11e and existing at a bending part of the flow path 22e branching toward one direction, a micro flow path 22f extending therefrom, a fluid-sample-delivering hole 23a at a terminal part thereof and a fluid-sample-receiving hole 21g at a terminal part of the micro flow path 22g branching toward the other direction while piercing the both faces thereof. Further, the flow-path-supporting substrate sheet 20 has a micro flow path 22i extending from a fluid-sample-receiving hole 21i and a fluid-sample-delivering hole 23b at a terminal part thereof while piercing the both faces thereof.

The flow-path-retaining substrate sheet 30 has fluid-sample-receiving holes 31c, 31 d, 31h, 31i corresponding to each of the fluid-sample-receiving holes 21c, 21d, 21g, 21i while piercing the both faces thereof.

The flow-path-supporting substrate sheet 40 has a fluid-sample-receiving hole 41c corresponding to the fluid-sample-receiving hole 31c, a micro flow path 42c which is extended therefrom and is bent halfway and a fluid-sample-receiving hole 41d at a terminal part thereof and corresponding to the fluid-sample-receiving hole 31d while piercing the both faces thereof. In addition, a fluid-sample-receiving hole 41 h corresponding to the fluid-sample-receiving hole 31 h, a micro flow path 42h extending therefrom and bending into an approximate Ω shape halfway, and a fluid-sample-receiving hole 41i at a terminal part thereof and corresponding to the fluid-sample-receiving hole 31i are pierced in the both faces of the flow-path-supporting substrate sheet 40.

A first face 54a of the flow-path-retaining substrate sheet 50 is flat.

The flow-path-retaining substrate sheets 10, 30, 50 are harder than the silicone-rubber-made flow-path-supporting substrate sheets 20, 40 and do not have rubber elasticity. Thus the flow-path-retaining substrate sheets 10, 30, 50 are not elastically deformed as distinct from the flow-path-supporting substrate sheets 20, 40. Because the flow-path-retaining substrate sheets 10, 30, 50 are thin sheets and have flexibility, these sheets may be comparably flexed so as to retain the flow-path-supporting substrate sheets 20, 40 and prevent peeling or removing of them.

The flow path is defined by the fluid-sample-injecting holes 11, the fluid-sample-receiving holes 21, 31, 41, the micro flow paths 22, 42, the fluid-sample-delivering holes 23 and the fluid-sample-draining holes 13.

The micro flow paths 22, 42 have channel shape, and progress chemical reactions by flowing a pressurized fluid sample selected from the group consisting of a liquid or gaseous specimen, reagent and sample thereinto. The micro flow paths 22, 42 are defined in the flow-path-retaining substrate sheets 10, 30 and the flow-path-supporting substrate sheets 20, 40. The micro flow paths 22, 42 have the channel shape or pore shape by piercing these sheets. The fluid sample is injected from the fluid-sample-injecting holes 11 to the micro flow paths 22, 42, induced to another micro flow paths 22, 42 of the flow-path-supporting substrate sheet 20, 40 and drained from the fluid-sample-draining holes 13 by each of the fluid-sample-injecting holes 11, the fluid-sample-receiving holes 21, 31, 41, the fluid-sample-delivering holes 23 and the fluid-sample-draining holes 13.

The flow-path-retaining substrate sheets 10, 30, 50 and the flow-path-supporting substrate sheets 20, 40 are sequentially joined each other and integrated. Each of second faces 14b, 24b, 34b, 44b of the flow-path-retaining substrate sheets 10, 30 and the flow-path-supporting substrate sheets 20, 40, and each of first faces 24a, 34a, 44a, 54a of the flow-path-retaining substrate sheets 30, 50 and the flow-path-supporting substrate sheets 20, 40 are joined each other. After the faces of the flow-path-retaining substrate sheet 10, 30, 50 and the flow-path-supporting substrate sheet 20, 40 are activated by a corona treatment, plasma treatment or ultraviolet irradiation treatment (a usual UV treatment and excimer UV treatment) except regions of the fluid-sample-injection holes 11, the fluid-sample-receiving holes 21, 31, 41, the fluid-sample-delivering holes 23 and the fluid-sample-draining holes 13, these sheets are stacked.

The flow-path-retaining substrate sheets 10, 30, 50 and the flow-path-supporting substrate sheets 20, 40 are integrated so as to be not removed each other. The integration is originated from a strong join of direct chemical bonds whereby a covalent bond is formed between active groups e.g. reactive active groups on the faces of these sheets. The reactive active groups are exemplified by the hydroxy group (-OH) and the hydroxysilyl group (-SiOH) which generate by the corona treatment, plasma treatment or ultraviolet irradiation treatment (the usual UV treatment and excimer UV treatment) to the faces of these sheets except the regions of the micro flow paths 22, 42, the fluid-sample-receiving holes 21 and the fluid-sample-delivering holes 23. A preferred ether bond is generated by dehydration between the OH groups.

The flow-path-supporting substrate sheets 20, 40 are made from a composite including a silicone rubber raw material component and formed.

A main component of the silicone rubber in the flow-path-supporting substrate sheet 20, 40 may be peroxide crosslinking type silicone rubber, addition crosslinking type silicone rubber and condensation crosslinking type silicone rubber or blended rubber made from the rubber and olefin type rubber.

All or any one of the flow-path-retaining substrate sheets 10, 30, 50 and the flow-path-supporting substrate sheets 20, 40 is a silicone rubber-made thermally conductive sheet including thermally conductive filler powder of at least one selected from the group consisting of aluminum oxide, magnesium oxide, zinc oxide, graphite carbon, silicon nitride, boron nitride and aluminum nitride. Each of the flow-path-retaining substrate sheets 10, 30, 50 and the flow-path-supporting substrate sheets 20, 40 includes 50 to 95% by weight of the thermally conductive filler powder. An average particle dimeter of the thermally conductive filler powder is preferably 0.2 to 50 µm.

A platinum catalyst from 10 to 1000 ppm concentration in platinum conversion is preferably contained into all or any one of the flow-path-supporting substrate sheet 20, 40 so that the active groups, which are generated by the corona treatment, plasma treatment and ultraviolet irradiation treatment (the usual UV treatment and excimer UV treatment), are easily bonded therebetween by the covalent bond. The platinum catalyst is exemplified by a platinum complex such as a platinum(0)-1,3-divinyl-1,1,3,3-tetramethyldisiloxane catalyst (Pt (dvs)) in 2.1 to 2.4% xylene solution (manufactured by Gelest Inc.).

A silane-coupling agent including 2-6 vinylalkoxysilane units having a vinylalkoxy group e.g. polyvinylmethoxysiloxane of 0.5 to 10 parts by weight of concentration is preferably included into all or any one of the flow-path-supporting substrate sheets 20, 40. Thereby the vinyl group of the silane-coupling agent, and the vinyl group in silicone rubber polymer and/or the hydrogen siloxane group may be more strongly joined by a covalent bond. This covalent bond is different with the ether bond of the covalent bond formed by the peroxide and the platinum catalyst. In this case, when the platinum catalyst is preferably included, the covalent bond may be easily formed.

In order to give function of flame resistance, powder of antimony trioxide and/or aluminum hydroxide, which exhibits action of the flame resistance, may be contained in all or any one of the flow-path-retaining substrate sheets 10, 30, 50 and the flow-path-supporting substrate sheets 20, 40. Concentration and an average particle diameter of these powders are preferably 5 to 50% by weight and 5 to 20 µm in the flow-path-retaining substrate sheets 10, 30, 50 and the flow-path-supporting substrate sheets 20, 40.

0.5 to 30% by weight of silicone oil may be contained in all or any one of the flow-path-retaining substrate sheets 10, 30, 50 and the flow-path-supporting substrate sheets 20, 40. The silicone oil is exemplified by polydimethylsiloxane, methylphenylsiloxane and fluorosilicone oil. When the silicone oil is added into the silicone rubber, the filler may be easily added thereinto.

All or any one of the flow-path-supporting substrate sheets 20, 40 may be made from foamable silicone rubber so that a refractive index is improved and function of a valve is exhibited. The foamable silicone rubber is made from a silicone rubber composite into which glass beans and/or zeolite is blended and a silicone rubber composite including an foamable silicone rubber component exemplified by a silicone composite containing water soluble alcohol.

All or any one of the flow-path-supporting substrate sheets 20, 40 may be made from a silicone rubber composite containing a high reflective index exhibition component and may be formed. The high reflective index exhibition component may be exemplified by a silicone composite including titanium oxide. Anatase-type or rutile-type titanium oxide is dispersed into the silicone rubber composite. Thereby, since these sheets exhibit the high reflective index, sensitive detection can be conducted by using a ray from fluorescent detection etc. The reflective index of the flow-path-supporting substrate sheets 20, 40 can be achieved in 80 to 100%.

As specific examples of the titanium oxide particles, rutile-type titanium oxide particles and anatase-type titanium oxide particles are included. In particular, the rutile-type titanium oxide particles are preferred. Because the rutile-type titanium oxide particles have low photocatalytic activity, decrease of the reflective index of resin by deterioration of the resin caused by the photocatalytic action may be inhibited. Further, the rutile-type titanium oxide is preferred from the point of exhibiting the high reflective index in long wavelength band of 500 nm or more. The anatase-type titanium oxide is preferred from the point of exhibiting the high reflective index in long wavelength band of 400 nm or more. Furthermore, the titanium oxide particles may be treated by a surface treatment to inhibit the photocatalytic activity. As an agent used in the surface treatment, zinc oxide, silica, alumina and zirconia are optionally selected.

In addition, the titanium oxide particles may be treated with the silane-coupling agent. When the titanium oxide particles are treated with the silane-coupling agent, dispersion properties thereof are improved and adhesive strength in boundary face with silicone resin is improved. As specific examples of the silane-coupling agent, a silane-coupling agent having reactive functional groups such as a vinyl group, phenyl group, alkoxy group, glycidyl group and (meth)acryloyl are exemplified. In particular, for example, CH₂=CHSi(OCH₃)₃ (vinylmethoxysilane), C₆H₅Si(OCH₃)₃, C₂H₃O-CH₂O(CH₂)₃Si(OCH₃)₃, C₂H₃O-CH₂O(CH₂)₃SiCH₃(OCH₃)₂, CH₂=CH-CO-O(CH₂)₃SiCH₃(OCH₃)₂, CH₂=CCH₃-CO-O(CH₂)₃SiCH₃(OCH₃)₂, 2-(2,3-epoxypropyloxypropyl)-2,4,6,8-tetramethyl-cyclotetrasiloxane, 2-(2,3-epoxypropyloxypropyl)-2,4,6,8-tetramethyl-6-(trimethoxysilylethyl)cyclotetrasi loxane are exemplified.

Although he examples of the flow-path-supporting substrate sheets 20, 40 made from the silicone rubber are mentioned above, these sheets may be made from other rubbers. All or any one of the flow-path-supporting substrate sheets 20, 40 may be made from silicone rubber, ethylene-propylene-diene-methylene copolymer rubber (EPDM), butyl rubber, acrylonitrile-butadiene copolymer rubber (NBR), fluoro-rubber, styrene-butadiene copolymer rubber (SBR) and/or hydrin rubber so as to avoid external action such as oxygen, carbon dioxide, water vapor (moisture) and the like by having low gas permeability. Specifically, a silicone rubber composite blended with EPDM and scaly-shaped filler such as mica and talc, or another silicone rubber composite including butyl rubber, and a silicone rubber composite including a low gas permeation exhibition component exemplified by the butyl rubber, NBR, fluoro-rubber, SBR and hydrin rubber are included. Thereby the flow-path-supporting substrate sheets 20, 40 may be a low-gas-permeable silicone rubber sheet having 500 to 0.05 (cc/cm²/mm/sec/cm·Hg×10¹⁰) of gas permeability of at least any gas of oxide gas, nitrogen gas, carbon dioxide gas and water vapor.

Although the examples of the flow-path-retaining substrate sheets 10, 30, 50 made from polyimide are mentioned above, these sheets may be made from cycloolefin polymer (COP) or aluminum foil or an aluminum plate and glass plate, may be made from the silicone exemplified by the materials for the flow-path-supporting substrate sheets 20, 40 and may include the flame retardant.

The low-path-retaining substrate sheets 10, 30, 50 and the flow-path-supporting substrate sheets 20, 40 may be joined and integrated by the covalent bond through a molecular adhesive.

According to the molecular adhesive, since functional groups in the molecule thereof are chemically reacted with an adhesive body by forming the covalent bond, the low-path-retaining substrate sheets 10, 30, 50 and the flow-path-supporting substrate sheets 20, 40 are directly bonded through the covalent bond by single molecule or multiple molecules of the molecular adhesive molecules. The two functional groups of the molecular adhesive forms the covalent bond by chemically reacting each with the low-path-retaining substrate sheets 10, 30, 50 and the flow-path-supporting substrate sheets 20, 40 of the bodies being adhered. Molecules having ambifunctionality collectively means the molecular adhesive, and specifically various coupling agents including the silane-coupling agents are exemplified.

As the molecular adhesive, more specifically,
a compound having an amino group such as triethoxysilylpropylamino-1,3,5-triazine-2,4-dithiol (TES), aminoethylaminopropyltrimethoxy silane;
a triazine compound having a trialkoxysilylalkylamino group such as the triethoxysilylpropylamino group, a mercapto group or an azide group, a triazine compound represented by Formula (I) as following 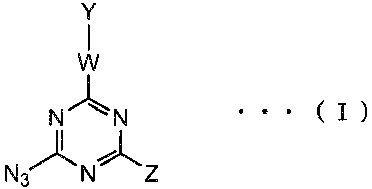 wherein W is a spacer group e.g. may be the alkylene group, aminoalkylene group which optionally have a substituted group or is directly bonded;
Y is an OH group or a reactive group which generates the OH group by hydrolysis or cleavage e.g. the trialkoxyalkyl group;
-Z is -N₃ or -NR¹R² (R¹ and R² are the same or different, H or an alkyl group, -R³Si(R⁴)ₘ(OR**⁵**)₃₋ₘ [R³ and R⁴ are an alkyl group, R⁵ is H or an alkyl group, m is 0 to 2]),
incidentally, the alkylene group, alkoxy group and alkyl group are the chained, branched and/or cyclic hydrocarbon group having 1 to 12 carbon atoms which optionally has a substituted group;
a thiol compound having a trialkoxysilylalkyl group;
an epoxy compound having a trialkyloxysilylalkyl group;
a silane-coupling agent such as a vinylalkoxysiloxane polymer exemplified by CH₂=CH-Si(OCH₃)₂-O-[Si(OCH₃)₂-O-]ₙ-Si(OCH₃)₂-CH=CH₂ (n=1.8 to 5.7)
are included.

When the flow-path-retaining substrate sheets 10, 30, 50 and the flow-path-supporting substrate sheets 20, 40 are made from the silicone rubber, the active group can be generated enough by only the corona treatment, plasma treatment and ultraviolet irradiation treatment (the usual UV treatment and excimer UV treatment) and thus, these sheets may be directly joined. These sheets may be joined by using the molecular adhesive such as the silane-coupling agent mentioned above. When the flow-path-retaining substrate sheets 10, 30, 50 are made from the resin of non-silicone rubber, they are preferably joined after being dipped into 0.05 to 1% by weight alcohol e.g. methanol of the molecular adhesive such as the silane-coupling agent and dried. If concentration of the molecular adhesive solution is excessive, the joined faces between the sheets are removed. If the concentration of the molecular adhesive solution is insufficient, both sheets cannot be sufficiently joined.

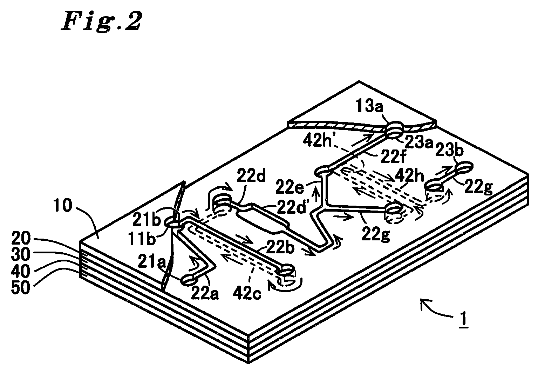

As shown in Fig. 2, the three-dimensional microchemical chip 1 is composed by the sequential join and integration of the flow-path-retaining substrate sheets 10, 30, 50 and the flow-path-supporting substrate sheets 20, 40. Thereby the micro flow paths 22, 42 defined in the plural flow-path-supporting substrate sheets 20, 40 are folded back halfway while sterically and sequentially connecting from the fluid-sample-injecting holes 11 to the fluid-sample-draining holes 13.

When the micro flow paths 22, 42 which form the flow path are only folded back at a part thereof, a pattern thereof is not restricted. As the pattern of the micro flow path 22, 42, a pattern for microanalysis by flowing a biological component in a test sample originated from a biological object thereinto, a pattern for chemical microsynthesis of a useful substance by flowing a raw material component of the useful substance such as the biological component exhibiting pharmacological action and a reagent of reactive substrate thereinto, and a pattern for multiplying useful cells and virus by accumulation of the cells and virus, or attaching cancer cells for an examination are exemplified.

According to the embodiment shown in Fig. 2, the flow path will be explained as follows. The flow path, which is started from the fluid-sample-injecting hole 11a of the flow-path-retaining substrate sheet 10, reaches the micro flow path 22a in the flow-path-supporting substrate sheet 20 as a lower face sheet through the fluid-sample-receiving hole 21 a. The fluid-sample-injecting hole 11b of the flow-path-retaining substrate sheet 10 meets the fluid-sample-receiving hole 21 b of the terminal of the micro flow path 22a. The fluid-sample-receiving hole 21b connects to the micro flow path 22b. The micro flow path 22b connects to the fluid-sample-receiving hole 21c of the terminal thereof. The fluid-sample-receiving hole 21c connects to the fluid-sample-receiving hole 41c of the flow-path-supporting substrate sheet 40 through the fluid-sample-receiving hole 31c of the flow-path-retaining substrate sheet 30. In the flow-path-supporting substrate sheet 40, the fluid-sample-receiving hole 41c reaches the fluid-sample-receiving hole 41 d as the terminal of the micro flow path 42c therethrough. The fluid-sample-receiving hole 41 d connects to the fluid-sample-receiving hole 21 d so as to go back to the flow-path-supporting substrate sheet 20 through the fluid-sample-receiving hole 31 d of the flow-path-retaining substrate sheet 30. The flow path therefore is folded back halfway from the fluid-sample-injecting hole 11a of the upstream flow path to the fluid-sample-receiving hole 21d of the downstream flow path. In the flow-path-supporting substrate sheet 20, the fluid-sample-receiving hole 21 d reaches the micro flow path 22d. The fluid accumulation part 22d' of the midway point of the micro flow path 22 is expanded at the midway point thereof so as to accumulate the fluid sample. The fluid sample which flows into the fluid accumulation part 22d' is accumulated therein for a short period. The chemical reaction of the fluid sample can be progressed enough thereat. In addition, the fluid accumulation part 22d' has a sufficiently specious area and thus, a ray for measurement, detection or reaction of the fluid sample can be irradiated thereto. As the ray, ultraviolet radiation, infrared radiation, visible radiation and laser radiation are exemplified. In the fluid accumulation part 22d', an accumulation starting edge part and accumulation ending edge part are each gradually expanded and then contracted. Thereby flow of the fluid sample is not inhibited. When the fluid sample is flowed into the fluid accumulation part 22d', the flow of the fluid sample to the micro flow path 22 by pressurization is stopped. The fluid sample is optionally subjected to heat, cool or radiate heat and allowed to react enough e.g. PCR. The three-dimensional microchemical chip may be programed so as to resume the flow of the fluid sample thereafter. The micro flow path 22d is bent ahead of the fluid accumulation part 22d' toward branching point. Herein, in the flow-path-supporting substrate sheets 20, 40, the micro flow path 22b and the micro flow path 42c are paralleled so as to overlap one above each other at a straight section. The micro flow path 22b and the micro flow path 22d are lined and paralleled at the straight sections thereof each other. Thereby the micro flow path can be allowed for a further distance. The micro flow path 22d is branched to the micro flow paths 22e, 22g in the flow-path-supporting substrate sheet 20.

The fluid-sample-injecting hole 11e of the flow-path-retaining substrate sheet 10 is joined to the fluid-sample-receiving hole 21e of a terminal part of the micro flow path 22e as one flow path which is branched. The flow path is reached therefrom to the micro flow path 22f and connected to the fluid-sample-delivering hole 23a of a terminal part thereof. The fluid-sample-delivering hole 23a is connected to the fluid-sample-draining hole 13a of the flow-path-retaining substrate sheet 10 so as to go back to upper face sheet.

The fluid-sample-injecting hole 11g of the flow-path-retaining substrate sheet 10 is joined to the fluid-sample-receiving hole 21g of a terminal part of the micro flow path 22g of the other flow path which is branched. The flow path is connected to the fluid-sample-receiving hole 41h of the flow-path-supporting substrate sheet 40 through the fluid-sample-receiving hole 31h of the flow-path-retaining substrate sheet30. The micro flow path 42, which is extended from the fluid-sample-receiving hole 41 h, is bent into an approximate Ω shape in the flow-path-supporting substrate sheet 40 and thus, the micro flow path can be allowed for a further distance. The micro flow path 42h', which is bent at a midway part of the approximate Ω shape, is paralleled with the flow path 22f of the flow-path-supporting substrate sheet 20 so as to overlap one above therewith at straight sections each other. By measuring transmittance and absorbance, comparison of strengths can be conducted at the overlapping section and the not overlapping section in the micro flow path 22f and the micro flow path 42h' as needed. The micro flow path 42h is connected to the fluid-sample-receiving hole 41 i of a terminal thereof. Thereby the flow path is folded back between the fluid-sample-receiving hole 41 h of upstream and the fluid-sample-receiving hole 41i of downstream. The micro flow path, which is extended from the fluid-sample-receiving hole 41 i, is connected to the fluid-sample-receiving hole 21 i of the flow-path-supporting substrate sheet 20 through the fluid-sample-receiving hole 31 i of the flow-path-retaining substrate sheet 30 so as to go back. The fluid-sample-receiving hole 21i is connected to the micro flow path 22i. The micro flow path 22i is reached to the fluid-sample-delivering hole 23b of a terminal thereof. The fluid-sample-delivering hole 23b is connected to fluid-sample-draining hole 13b of the flow-path-retaining substrate sheet 10. Thus, the flow path is sequentially and sterically connected from the fluid-sample-injecting holes 11 to the fluid-sample-draining holes 13.

The patterns of the micro flow paths 22, 42 of the flow path may be folded back from a draining side B toward an injecting side A or paralleled with the injecting and draining sides A, B. The folded back part may be regularly continued or intermittently and plurally repeated.

When the flow-path-retaining substrate sheets 10, 30, 50 and the flow-path-supporting substrate sheets 20, 40 are joined and integrated, these bonded faces may be conducted the corona treatment, plasma treatment or ultraviolet treatment (the usual UV treatment and excimer UV treatment), and these sheets may be stacked under conditions of normal atmospheric pressure and bonded by the covalent bond under the conditions thereof. These sheets may be bonded by the covalent bond under conditions of pressurization or reduced pressure. Approach of the active groups such as OH of the flow-path-retaining substrate sheets 10, 30, 50 and the flow-path-supporting substrate sheets 20, 40 or the reactive functional groups of the silane-coupling agent, which reacts therewith, is accelerated by removing gaseous media of contact boundaries under conditions of the reduced pressure or a vacuum conditions, for example, 50 torr or less, more particularly, the reduced pressure conditions of 50 to 10 torr, or the vacuum conditions of less than 10torr, more particularly, less than 10torr to 1×10⁻³ torr, preferably less than 10torr to 1×10⁻² torr, or by adding a stress (a load) e.g. 10 to 200 kgf to the contact boundaries thereof, and further by heating the contact boundaries thereof. The whole surfaces of the bonded faces of the flow-path-retaining substrate sheets 10, 30, 50 and the flow-path-supporting substrate sheets 20, 40 are preferably homogeneously pressurized. If the values fall outside the above range, the pressure could not homogeneously be applied.

The single or plural flow-path-retaining substrate sheets 10, 30, which support the single or plural flow-path-supporting substrate sheet 20 while contacting the most upper face and/or most lower face thereof, and the flow-path-supporting substrate sheet 20 are stacked, joined and integrated at the surface of at least any one of the sheets 10, 20, 30 by a direct covalent bond through at least any one of a dry treatment selected from the group consisting of the corona treatment, plasma treatment and ultraviolet irradiation treatment (the usual UV treatment and excimer UV treatment), and a molecular adhesive treatment and/or by an indirect covalent bond via the molecular adhesive. In order to join, the dry treatment and the molecular adhesive treatment may be conducted only any one of these treatments, and alternately and contiguously conducted these treatments. For example, joining of these sheets may be conducted by only the dry treatment, by the molecular adhesive treatment following the dry treatment, by and the molecular adhesive treatment following the dry treatment and then the additional dry treatment, by only the molecular adhesive treatment, by the dry treatment the molecular adhesive treatment following the molecular adhesive treatment or by the dry treatment following the molecular adhesive treatment and the additional molecular treatment.

A rubber component of the flow-path-retaining substrate sheets 10, 30, 50 and the flow-path-supporting substrate sheet 20, 40 may consist of the silicone rubber, or may include the non-silicone rubber. Particularly, the flow-path-retaining substrate sheets 10, 30, 50 and the flow-path-supporting substrate sheet 20, 40 may be made from the silicone rubber exemplified by peroxide crosslinking type silicone rubber, addition crosslinking type silicone rubber and condensation crosslinking type silicone rubber; three-dimensional silicone rubber exemplified by blended rubber of such silicone rubber mentioned above with olefin rubber. These sheets may be a silicone rubber elastic sheet, which is made from these rubbers by molding or stretching, and optionally crosslinking. These rubber raw materials have a number average molecular weight from ten thousand to one million.

The peroxide crosslinking type silicone rubber, which is a raw material for the flow-path- retaining substrate sheets 10, 30, 50 and the flow-path-supporting substrate sheet 20, 40, is not specifically limited as far as the rubber synthesized from a silicone raw compound and crosslinked by a peroxide type crosslinking agent. Particularly, polydimethylsiloxane, vinylmethylsiloxane/polydimethylsiloxane copolymer, vinyl-terminated polydimethylsiloxane, vinyl-terminated diphenylsiloxane/polydimethylsiloxane copolymer, vinyl-terminated diethylsiloxane/polydimethylsiloxane copolymer, vinyl-terminated trifluoropropylmethylsiloxane/polydimethylsiloxane copolymer, vinyl-terminated polyphenylmethylsiloxane, vinylmethylsiloxane/dimethylsiloxane copolymer, trimethylsiloxane group-terminated dimethylsiloxane/vinylmethylsiloxane copolymer, trimethylsiloxane group-terminated dimethylsiloxane/vinylmethylsiloxane/diphenylsiloxane copolymer, trimethylsiloxane group-terminated dimethylsiloxane/vinylmethylsiloxane/ditrifluoropropylmethylsiloxane copolymer, trimethylsiloxane group-terminated polyvinylmethylsyloxane, methacryloxypropyl group-terminated polydimethylsiloxane, acryloxypropyl group-terminated polydimethylsiloxane, (methacryloxypropyl)methylsiloxane/dimethylsiloxane copolymer, and (acryloxypropyl)methylsiloxane/dimethylsiloxane copolymer may be exemplified.

As the peroxide type crosslinking agent which is coexisted therewith, for example, ketone peroxides, diacyl peroxides, hydroperoxides, dialkylperoxides, peroxyketals, alkylperesters, percarbonates may be exemplified. More particularly, ketoneperoxide, peroxyketal, hydroperoxide, dialkylperoxide, peroxycarbonate, peroxyester, benzoylperoxide, dicumylperoxide, dibenzoylperoxide, tert-butylhydroperoxide, di-tert-butylhydroperoxide, di(dicyclobenzoyl)peroxide. 2,5-dimethyl-2,5-bis(tert-butylperoxy)hexane,
2,5-dimethyl-2,5-bis(tert-butylperoxy)hexyne, benzophenone, Michler's ketone, dimethylaminobenzoic acid ethyl ester and benzoin ethyl ether may be exemplified.

The amount to be used as the peroxide type crosslinking agent can be arbitrarily determined depending on properties of the silane-coupling agent which is optionally used, and properties of the flow-path-retaining substrate sheets 10, 30, 50 and the flow-path-supporting substrate sheet 20, 40 which is made from the silicone rubber or kinds of the silicone rubber prepared. As the peroxide type crosslinking agent, 0.01 to 10 parts by mass, more preferably 0.1 to 2 parts by mass relative to 100 parts b) mass of silicone rubber can be preferably used. If the amount is less than this range, crosslink density is excessively low to give undesired properties as the silicone rubber. If the amount is more than this range, crosslink density is excessively high, and elasticity of the silicone rubber is decreased.

The addition type silicone rubber which is a raw material for the flow-path-retaining substrate sheets 10, 30, 50 and the flow-path-supporting substrate sheet 20, 40 can be obtained by synthesis using below composites in the presence of a Pt catalyst. As the composites, a composite containing polysiloxanes having a vinyl group and polysiloxanes having a H group is included. As polysiloxane having the vinyl group, vinylmethylsiloxane/polydimethylsiloxane copolymer, vinyl-terminated polydimethylsiloxane, vinyl-terminated diphenylsiloxane/polydimethylsiloxane copolymer, vinyl-terminated diethylsiloxane/polydimethylsiloxane copolymer, vinyl-terminated trifluoropropylmethylsiloxane/polydimethylsiloxane copolymer, vinyl terminated polyphenylmethylsiloxane, vinylmethylsiloxane/dimethylsiloxane copolymer, trimethylsiloxane group-terminated dimethylsiloxane/vinylmethylsiloxane/diphenylsiloxane copolymer, trimethylsiloxane group-terminated dimethylsiloxane/ vinylmethylsiloxane/ditrifluoropropylmethylsiloxane copolymer and trimethylsiloxane group-terminated polyvinylmethylsiloxane are included. As polysiloxane having the H group, H-terminated polysiloxane, methyl H siloxane/dimethylsiloxane copolymer, polymethyl H siloxane, polyethyl H siloxane, H-terminated polyphenyl(dimethyl H siloxy)siloxane, methyl H siloxane/phenylmethylsiloxane copolymer and methyl H siloxane/octylmethylsiloxane copolymer are included.

As other composites for synthesis of the addition type silicone rubber, a composite containing polysiloxanes having an amino group, and polysiloxanes having an epoxy group, polysiloxanes having an acid anhydride group or compounds having an isocyanato group. As polysiloxanes having the amino group, aminopropyl-terminated polydimethylsiloxane, aminopropylmethylsiloxane/dimethylsiloxane copolymer, aminoethylaminoisobutylmethylsiloxane/dimethylsiloxane copolymer, aminoethylaminopropylmethoxysiloxane/dimethylsiloxane copolymer and dimethylamino-terminated polydimethylsiloxane are included. As polysiloxanes having the epoxy group, epoxypropyl-terminated polydimethylsiloxane and (epoxycyclohexylethyl)methylsiloxane/dimethylsiloxane copolymer are included. As polysiloxanes having the acid anhydride group, succinic acid anhydride-terminated polydimethylsiloxane is included. As compounds having the isocyanato group, toluyldiisocyanate and 1,6-hexamethylene diisocyanate are included.

Processing conditions to prepare the flow-path-retaining substrate sheets 10, 30, 50 and the flow-path-supporting substrate sheet 20, 40 from these composities cannot be determined unambiguously because the processing conditions vary with the kinds and characteristics of addition reactions, but generally the preparation can be carried out at 0 to 200°C for 1 minute to 24 hours. Under these conditions, the addition type silicone rubber can be obtained as the flow-path-retaining substrate sheets 10, 30, 50 and the flow-path-supporting substrate sheet 20, 40. In cases where preparation is carried out at a low temperature to obtain a silicone rubber having good physical properties, the reaction time should be lengthened. In cases where productivity is more emphasized rather than the physical properties, the preparation should be carried out at a higher temperature for a shorter period of time. If the preparation should be carried out within a certain period of time in compliance with the production processes or working conditions, the preparation should be carried out at a comparatively higher temperature within the range to meet a desired period of processing time.

The condensation type silicone rubber of material for the flow-path-retaining substrate sheets 10, 30, 50 and the flow-path-supporting substrate sheet 20, 40 can be obtained by synthesis using below composites. The composites of a homocondensation component consisting of silanol group-terminated polysiloxanes which is prepared in the presence of a tin catatlyst, a composite containing these silanol group-terminated polysiloxanes and crosslinking agents, and a composite containing these silanol group-terminated polysiloxanes, and terminal-blocked polysiloxanes exemplified by chloro-terminated polydimethylsiloxane, diacetoxymethyl-terminated polydimethylsiloxane and terminal-blocked polysiloxane.

As silanol group-terminated polysiloxanes, silanol-terminated polydimethyl siloxane, silanol-terminated polydiphenylsiloxane, silanol-terminated polytrifluoromethylsiloxan and silanol-terminated diphenyl siloxane/dimethylsiloxane copolymer are included.

As the crosslinking agents, tetraacetoxysilane, triacetoxymethylsilane, di t-butoxydiacetoxysilane, vinyltriacetoxysilane, tetraethoxysilane, triethoxymethylsilane, bis(triethoxysilyl)ethane, tetra-n-propoxysilane, vinyltrimethoxysilane, methyltris(methylethylketoxim)silane, vinyltris(methylethylketoximino)silane, vinyltriisopropenoxysilane, triacetoxymethylsilane, tri(ethylmethyl)oximmethylsilane, bis(N-methylbenzoamido)ethoxymethylsilane, tris(cyclohexylamino)methylsilane, triacetoamidomethylsilane and tridimethylamino methylsilane are included.

Processing conditions to prepare the condensation type silicone rubbers from these composites cannot be determined unambiguously because the processing conditions vary according to the kinds and characteristics of condensation reactions, but generally the preparation can be carried out at 0 to 100°C for 10min. to 24 hours. Under these conditions, the condensation type silicone rubbers can be obtained as the flow-path-retaining substrate sheets 10, 30, 50 and the flow-path-supporting substrate sheet 20, 40. In cases where the preparation is carried out at a low temperature to obtain a silicone rubber having good physical properties, the reaction time should be lengthened. In cases where productivity is more emphasized rather than the physical properties, the preparation should be carried out at a higher temperature for a shorter period of time. If the preparation should be carried out within a certain period of time in compliance with production processes or working conditions, the preparation should be carried out at a comparatively higher temperature within the range to meet a desired period of processing time.

The blended rubber material for the flow-path-retaining substrate sheets 10, 30, 50 and the flow-path-supporting substrate sheet 20, 40 comprises the silicone rubber with the olefin rubber. As the olefin rubber, 1, 4-cis-butadiene rubber, isoprene rubber, styrene-butadiene copolymer rubber, polybutene rubber, polyisobutylene rubber, ethylene-propylene rubber, ethylene-propylene-diene rubber, chlorinated ethylene-propylene rubber and chlorinated butyl rubber may be exemplified.

The blended rubber material for the flow-path-retaining substrate sheets 10, 30, 50 and the flow-path-supporting substrate sheet 20, 40 comprises the silicone rubber with the non-silicone rubber to obtain them by crosslinking of the composite of raw material rubbers. As the non-silicone rubber, an ethylene-propylene-diene rubber, natural rubber, 1,4-cis butadiene rubber, isoprene rubber, polychloroprene, styrene-butadiene copolymer rubber, hydrogenated styrene-butadiene copolymer rubber, acrylonitrile-butadiene copolymer rubber, hydrogenated acrylonitrile-butadiene copolymer rubber, polybutene rubber, polyisobutylene rubber, ethylene-propylene rubber, ethylene oxide-epichlorohydrin copolymer rubber, chlorinated polyethylene rubber, chlorosulfonated polyethylene rubber, alkylated chlorosulfonated-polyethylene rubber, chloroprene rubber, chlorinated acryl rubber, brominated acryl rubber, fluoro rubber, epichlorohydrin rubber and its copolymer rubber, chlorinated ethylene propylene rubber, chlorinated butyl rubber, brominated butyl rubber, homopolymer rubber or two- or three- dimensional co- or ter- polymer rubber using such a monomer as tetrafluoroethylene, hexafluoropropylene, vinylidene fluoride and tetrafluoroethylene, ethylene/tetrafluoroethylene copolymer rubber, propylene/tetrafluoroethylene copolymer rubber, ethylene-acryl rubber, epoxy rubber, urethane rubber, liner polymer of both terminals unsaturated-group elastomer etc. may be exemplified. These rubbers may be used solely or mixture thereof.

When the diatomaceous earth, mica, talc and/or kaolin is included in all or any one of the low-path-retaining substrate sheets 10, 30, 50 and the flow-path-supporting substrate sheets 20, 40, content rate of the silicone rubber is decreased, and permeation of the water vapor is inhibited. Thereby water soluble liquid is difficult to permeate these sheets and to vaporize. Amount of the permeation of the liquid is decreased as additive amount of the diatomaceous earth, mica, talc and/or kaolin is increased. Further the above additive agent is more preferably shaped in the scaly-shape. In the case of the scaly-shape, pathway in these sheets, in which the water vapor is permeated, is allowed for a further distance and thus, the permeation of the water vapor may be inhibited.

All or any one of the low-path-retaining substrate sheets 10, 30, 50 and the flow-path-supporting substrate sheets 20, 40 is preferably made from the blended composite which includes the silicone rubber as a main component and the olefin rubber as the secondary component. As the silicone rubber, the peroxide crosslinking type silicone rubber, addition crosslinking type silicone rubber condensation crosslinking type silicone rubber or radiation or electron beam crosslinking silicone rubber are exemplified. As the olefin rubber, the ethylene-propylene-diene rubber (EPDM) is exemplified. Ratio of the main component and the secondary component is preferably 5 to 100: 100 to 5 parts by weight. The ethylene-propylene-diene rubber may be used alone. When the ethylene-propylene-diene rubber is used alone or it is blended (SEP), the water soluble liquid is difficult to permeate and vaporize.

The micro flow paths 22, 42 of the flow-path-supporting substrate sheets 20, 40 may have 0.5 µm to 5 mm, preferably 10 to 1000 µm in width, the especially non-restricted shape, any one of a straight line and curved line having a continuous liner shape and/or branched linear shape, and an arrangement of a singular or plural parallel. The flow-path-retaining substrate sheets 10, 30, 50 and the flow-path-supporting substrate sheets 20, 40 preferably have 5 to 100 µm in thickness. Since the micro flow paths 22, 42 have the narrow width and the flow-path-retaining substrate sheets 10, 30, 50 and the flow-path-supporting substrate sheets 20, 40 have the thin thickness, a contact area between the specimen, reagent and/or sample and the rubber sheets may be minimalized. Further, contamination of the specimen, reagent and/or sample due to leakage of a rubber component from the rubber sheet and an adsorption thereof to the rubber component may be prevented. In order to prevent the contamination and adsorption of the specimen reagent and/or sample, when at least a side surface of the micro flow paths 22, 42 is coated or deposited with a non-reactive resin or is deposited with a non-reactive inorganic substance, contact between the rubber sheet and the specimen reagent and/or sample can be completely avoided. Thereby, the contamination and adsorption of the specimen reagent and/or sample may be more prevented. As the non-reactive resin, a fluorine resin such as a polytetrafluoroethylene resin, a phosphoric resin such as 2-(methacryloyloxy)ethyl 2-(trimethylammonio)ethyl phosphate (MPC) polymer and a paraxylylene resin such as parylene are included. As the non-reactive inorganic substance, titanium dioxide and silicon dioxide are included. The fluid-sample-injecting holes 11, the fluid-sample-receiving holes 21, 31, 41, the fluid-sample-delivering holes 23 and the fluid-sample-draining holes 13 are in similar to the micro flow paths 22, 42. These holes have 0.5 µm to 5 mm in width or diameter, and a size equivalent to the micro flow paths 22, 42 or are comparably larger than it.

The fluid-sample-injecting holes 11, fluid-sample-receiving holes 21, 31, 41, the micro flow path 22, 42, the fluid-sample-delivering holes 23 and the fluid-sample-draining holes 13 are pierced by laser processing.

The three-dimensional microchemical chip 1 may be sandwiched by protecting-substrate sheets (not shown) which cover the flow-path-retaining substrate sheets 10, 50.

The protecting-substrate sheets may be made from a metal, ceramics, glass or resin. The protecting-substrate sheets may be formed into single-plate shape or thin-layer shape and they may be processed in laminate. The protecting-substrate sheets may be subjected to the various activation treatments in the same manner as the integration of the flow-path-retaining substrate sheets 10, 30, 50 and the flow-path-supporting substrate sheets 20, 40 each other by direct join thereof via the corona treatment, plasma treatment or ultraviolet irradiation treatment (the usual UV treatment or excimer UV treatment). Thereby the protecting-substrate sheets may be integrated to be the three-dimensional microchemical chip 1 by joining the flow-path-retaining substrate sheets 10, 50 at upper and lower sides thereof. Holes corresponding to the fluid-sample-injecting holes 11a, 11b, 11e, 11g of the flow-path-retaining substrate sheets 10 and the fluid-sample-draining holes 13a, 13b are opened into the protecting-substrate sheets. The protecting-substrate sheets are comparably stable against the specimen, reagent and sample, and sites contacting thereto are preferably made from resin, coated by a coating agent or processed in laminate.

As the metal, which is the material for the protecting-substrate sheets, metal such as gold, silver, copper, iron, cobalt, silicon, lead, manganese, tungsten, tantalum, platinum, cadmium, tin, palladium, nickel, chromium, titanium, zinc, aluminum, magnesium and a binary-, ternary- and multi-component metal alloys comprising of those metals may be exemplified.

As ceramics, which is the material for the protecting-substrate sheets, oxide, nitride, and carbide of metal such as silver, copper, iron, cobalt, silicon, lead, manganese. tungsten, tantalum, platinum, cadmium, tin, palladium, nickel, chromium, indium, titanium, zinc, calcium, barium, aluminum, magnesium, sodium, potassium etc. and a single or composite body thereof may be exemplified.

As a glass, which is the material for the protecting-substrate sheets, quartz, borosilicate glass and non-alkaline glass may be exemplified.

As a resin, which is the material for the protecting-substrate sheets, a resin such as polycarbonate resin,cycloolefin resin, acryl resin, epoxy resin, polyethylene terephthalate resin, polybutylene terephthalate resin, cellulose and derivatives thereof, hydroxyethyl cellulose, starch, diacetylcellulose, surface-saponified vinylacetate resin. low-density polyethylene, high-density polyethylene, i-polypropylene, petroleum resin, polystyrene, s-polystyrene, chromane-indene resin, terpene resin, styrene-divinylbenzene copolymer, ABS resin, polymethyl acrylate, polyethyl acrylate, polyacrylonitrile, polymethyl methacrylate, polyethyl methacrylate, polycyanoacrylate, polyvinyl acetate, polyvinyl alcohol, polyvinylformal, polyvinylacetal, polyvinyl chloride, vinyl chloride-vinyl acetate copolymer, vinyl chloride-ethylene copolymer, polyvinylidene fluoride, vinylidene fluoride-ethylene copolymer, vinylidene fluoride-propylene copolymer, 1,4-trans-polybutadiene, polyoxymethylene, polyethylene glycol, polypropylene glycol, phenol-formalin resin, cresol-formalin resin, resorcin resin, melamine resin, xylene resin, toluene resin, glyptal resin, modified glyptal resin, unsaturated polyester resin, allylester resin, 6-nylon, 6,6-nylon, 6,10-nylon, polyimide, polyamide, polybenzimidazole, polyamideimide, silicon resin, silicone rubber, silicone resin, furan resin, polyurethane resin, polyphenyleneoxide, polydimethylphenyleneoxide, mixture of triallyl isocyanurate compound with polyphenyleneoxide or polydimethylphenyleneoxide, mixture of (polyphenyleneoxide or polydimethylphenyleneoxide, triallyl isocyanurate, peroxide), polyxylene, polyphenylenesulfide (PPS), polysulfone (PSF), polyethersulfone (PES), polyether ether ketone (PEEK), polyimide (PPI, Kapton), polytetrafluroethylene (PTFE), liquid crystal resin, Kevlar fiber, carbon fiber, polymeric material exemplified by a mixture of a plurality of these resins and crosslinked products thereof may be exemplified.

When the joining faces between the protecting-substrate sheets and the flow-path-retaining substrate sheets 10, 50 are artificially activated, the corona treatment, plasma treatment or ultraviolet irradiation treatment (the usual UV treatment and excimer UV treatment) may be used. The protecting-substrate sheets made from the metal, ceramics, or glass and the flow-path-retaining substrate sheets 10, 50 are strongly joined through the ether bond produced by the dehydration of the active groups, e.g. between the hydroxy groups, which are generated by the activation treatment thereof. When the active groups such as the hydroxy groups are preliminarily exposed enough to form the ether bond by only stacking these sheets, the activation treatment is not required.

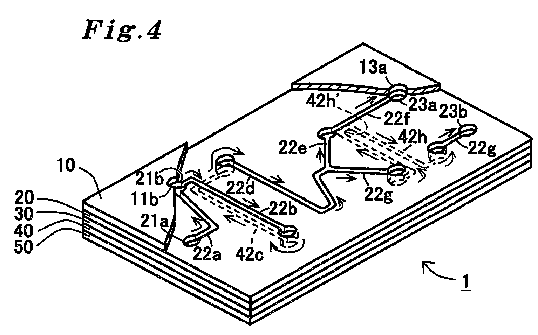

The embodiment of the three-dimensional microchemical chip 1 having the fluid accumulation part 22d' at the midway part of the micro flow path 22 is mentioned above. When the fluid sample can be reacted enough in the midway part of the flow path, the ray for the measurement, detection or reaction of the fluid sample can be irradiated enough to the micro flow path 22 or the irradiation of these rays is not required, the fluid accumulation part 22d' may have the same width with the micro flow path 22 as shown in Fig. 4. Incidentally, as the ray, ultraviolet radiation, infrared radiation, visible radiation and laser radiation are exemplified.

The examples of the direct bond between the protecting-substrate sheets and flow-path-retaining substrate sheet 10, 50 through the ether bond are mentioned above. The protecting-substrate sheets and the flow-path-retaining substrate sheet 10, 50 may be indirectly bonded by the chemical bond such as the covalent bond or hydrogen bond through the silane-coupling agent. In this case, a single molecule of the silane-coupling agent can form the chemical bond by mediating between the protecting-substrate sheets and the flow-path-retaining substrate sheet 10, 50. For example, at least any one of the joining faces between the flow-path-retaining substrate sheet 10, 50 and the protecting-substrate sheets made from the metal, ceramics, glass or resin are activated by the corona treatment, plasma treatment or ultraviolet irradiation treatment (the usual UV treatment and excimer UV treatment). The protecting-substrate sheets and the flow-path-retaining substrate sheet 10, 50 are joined through the chemical bond which is mediated by the silane-coupling agent having an amino group and/or alkoxy group having 1 to 4 carbons or an alkoxy equivalent group having hydrolyzability which may produce the ether bond by a reaction with the hydroxy group as well as these groups.

As the molecular adhesive, a compound having the amino group such as triethoxysilylpropylamino-1,3,5-triazine-2,4-dithiol (TES), aminoethylaminopropyltrimethoxy silane; a triazine compound having the trialkoxysilylalkylamino group such as the triethoxysilylpropylamino group and a mercapto group or an azide group, a triazine compound represented by Formula (I) as above e.g. 2,6-diazide-4-{3-(triethoxysilyl)propylamino}-1,3,5-triazine (P-TES); a thiol compound having the trialkoxysilylalkyl group; an epoxy compound having the trialkyloxysilylalkyl group are included.

In the molecular adhesive, as a silane-coupling agent having an alkoxy group without an amino group, an available silane-coupling agent is included. Particularly, a silane-coupling agent having a vinyl group and alkoxy group exemplified by vinylmethoxysilane (KBM-1003) and vinyltriethoxysilane (KBE-1003); a silane-coupling agent having an epoxy group and alkoxy group exemplified by 2-(3,4-epoxycyclohexyl)ethyltrimethoxysilane (KBM-303), 3-glycidoxypropyl methyldimethoxysilane (KBM-402), 3-glycidoxypropyl trimethoxysilane (KBM-403), 3-glycidoxypropyl methyldiethoxysilane (KBE-402), and 3-glycidoxypropyl triethoxysilane (KBE-403); a silane-coupling agent having a styryl group and alkoxy group exemplified by p-styryltrimethoxysilane (KBM-1403); a silane-coupling agent having a (meth)acryl group and alkoxy group exemplified by 3-methacryloxypropyl methyldimethoxysilane (KBM-502), 3-methacryloxypropyl methyldiethoxysilane (KBM-503), 3-methacryloxypropyl methyldiethoxysilane (KBE-502), 3-methacryloxypropyl triethoxysilane (KBE-503), 3-acryloxypropyl trimethoxysilane (KBM-5103); a silane-coupling agent having an ureido group and alkoxy group exemplified by 3-ureidopropyltriethoxysilane (KBE-585); a silane-coupling agent having a mercapto group and alkoxy group exemplified by 3-mercaptopropylmethyldimethoxysilane (KBM-802) and 3-mercaptopropyltrimethoxysilane (KBM-803); a silane-coupling agent having a sulfide group and alkoxy group exemplified by bis(triethoxysilylpropyl) tetrasulfide (KBE-846); and a silane-coupling agent having an isocyanate group and alkoxy group exemplified by 3-isocyanatepropyltriethoxysilane (KBE-9007) (all of which is manufactured by Shin-Etsu Chemical Co., Ltd.; trade names) may be exemplified. Further, a silane-coupling agent having a vinyl group and acetoxy group exemplified by vinyltriacetoxysilane (Z-6075); a silane-coupling agent having an allyl group and alkoxy group exemplified by allyltrimethoxysilane (Z-6285); a silane-coupling agent having an alkyl group and alkoxy group exemplified by methyltrimethoxysilane (Z-6366), dimethyldimethoxysilane (Z-6329), trimethylmethoxysilane (Z-6013), methyltriethoxysilane (Z-6383), methyltriphenoxysilane (Z-6721), ethyltrimethoxysilane (Z-6321), n-propyltrimethoxysilane (Z-6265), diisopropyldimethoxysilane (Z-6258), isobutyltrimethoxysilane (Z-2306), diisobutyldimethoxysilane (Z-6275), isobutyltriethoxysilane (Z-6403), n-hexyltrimethoxysilane (Z-6583), n-hexyltriethoxysilane (Z-6586), cyclohexylmethyldimethoxysilane (Z-6187), n-octyltriethoxysilane (Z-6341), and n-decyltrimethoxysilane (Z-6210); a silane-coupling agent having an aryl group and alkoxy group exemplified by phenyltrimethoxysilane (Z-6124); a silane-coupling agent having an alkyl group and chlorosilane group exemplified by n-octyldimethylchlorosilane (ACS-8); a silane-coupling agent of an alkoxysilane exemplified by tetraethoxysilane (Z-6697) (all of which is manufactured by Dow Corning Toray Co., Ltd.; trade names) may be exemplified.

As a silane-coupling agent having an alkoxy group without an amino group, an alkoxysilyl compound having the hydrosilyl group (a SiH group) may be exemplified. For example,
(CH₃O)₃SiCH₂CH₂CH₂Si(CH₃)₂OSi(CH₃)₂H,
(C₂H₅O)₃siCH₂CH₂CH₂Si(CH₃)₂OSi(CH₃)₂H,
(CH₃O)₃SiCH₂CH₂CH₂Si(OCH₃)₂OSi(OCH₃)₃,
(C₂H₅O)₃SiCH₂CH₂CH₂Si(OCH₃)₂OSi(OCH₃)₃,
(C₂H₅O)₃SiCH₂CH₂CH₂Si(CH₃)₂H,
(CH₃O)₃SiCH₂CH₂CH₂Si(CH₃)₂H,
(i-C₃H₇O)₃SiCH₂CH₂CH₂Si(CH₃)H₂,
(n-C₃H₇O)₃SiCH₂CH₂CH₂Si(CH₃)₂OSi(CH₃)₂CH₂CH₂Si(CH₃)₂Si(CH₃)₂H,
(n-C₄H₉O)₃SiCH₂CH₂CH₂Si(CH₃)₂OSi(CH₃)₂H,
(t-C₄H₉O)₃SiCH₂CH₂CH₂Si(CH₃)₂OSi(CH₃)₂H,
(C₂H₅O)₂CH₃SiCH₂CH₂CH₂Si(CH₃)₂OSi(CH₃)₂H,
(CH₃O)₂CH₃SiCH₂CH₂CH₂Si(CH₃)₂OSi(CH₃)₂CH₂CH₂Si(CH₃)₂Si(CH₃)₂H,
CH₃O(CH₃)₂SiCH₂CH₂CH₂Si(CH₃)₂OSi(CH₃)₂H,
(C₂H₅O)₃SiCH₂CH₂CH₂Si(CH₃)₂OSi(CH₃)₂H,
(n-C₃H₇)₃SiCH₂CH₂CH₂Si(CH₃)₂OSi(CH₃)₂H,
(i-C₃H₇O)₃SiCH₂CH₂CH₂Si(CH₃)₂OSi(CH₃)₂H,
(n-C₄H₉)₃SiCH₂CH₂CH₂Si(CH₃)₂OSi(CH₃)₂H,
(t-C₄H₉O)₃SiCH₂CH₂CH₂Si(CH₃)₂OSi(CH₃)₂H,
(C₂H₅O)₃SiCH₂CH₂Si(CH₃)₂OSi(CH₃)₂H,
(C₂H₅O)₃SiCH₂CH₂CH₂CH₂Si(CH₃)₂OSi(CH₃)₂H,
(C₂H₅O)₃SiCH₂CH₂CH₂CH₂CH₂CH₂Si(CH₃)₂OSi(CH₃)₂H,
(C₂H₅O)₃SiCH₂CH₂CH₂CH₂CH₂CH₂CH₂CH₂CH₂CH₂Si(CH₃)₂OSi(CH₃)₂H,
(CH₃O)₃SiCH₂C₆H₄CH₂CH₂Si(CH₃)₂C₆H₄Si(CH₃)₂H,
(CH₃O)₂CH₃SiCH₂C₆H₄CH₂CH₂Si(CH₃)₂C₆H₄Si(CH₃)₂H,
CH₃O(CH₃)₂SiCH₂C₆H₄CH₂CH₂Si(CH₃)₂C₆H₄Si(CH₃)₂H,
(C₂H₅O)₃SiCH₂C₆H₄CH₂CH₂Si(CH₃)₂C₆H₄Si(CH₃)₂H,
(C₂H₅O)₃SiCH₂CH₂CH₂Si(CH₃)₂C₆H₄OC₆H₄Si(CH₃)₂H,
(C₂H₅O)₃SiCH₂CH₂CH₂Si(CH₃)₂C₂H₄Si(CH₃)₂H,
(C₂H₅O)₃SiCH₂CH₂CH₂Si(CH₃)₂O[Si(CH₃)₂O]ₚ₁Si(CH₃)₂H,
C₂H₅O(CH₃)₂SiCH₂CH₂CH₂Si(CH₃)₂O[Si(CH₃)₂O]ₚ₂Si(C₂H₅)₂H,
(C₂H₅O)₂CH₃SiCH₂CH₂CH₂Si(CH₃)₂O[Si(CH₃)₂O]ₚ₃Si(CH₃)₂H,
(CH₃)₃SiOSiH(CH₃)O[SiH(CH₃)O]ₚ₄Si(CH₃)₃,
(CH₃)₃SiO[(C₂H₅OSi(CH₃)CH₂CH₂CH₂)SiCH₃]O[SiH(CH₃)O]ₚ₅Si(CH₃)₃,
(CH₃)₃SiO[(C₂H₅OSiOCH₃CH₂CH₂CH₂)SiCH₃]O[SiH(CH₃)O]ₚ₆Si(CH₃)₃,
(CH₃)₃SiO[(C₂H₅OSi(CH₃)CH₂CH₂CH₂)SiCH₃]O[SiH(CH₃)O]ₚ₇Si(CH₃)₃,
(CH₃)₃SiO[(Si(OC₂H₅)₂CH₂CH₂CH₂)SiCH₃]O[SiH(CH₃)O]ₚ₈Si(CH₃)₃,
(CH₃)₃SiOSi(OC₂H₅)₂O[SiH(CH₃)O]ₚ₉[Si(CH₃)₂O]_{q1}Si(CH₃)₃,
(CH₃)₃SiO[(C₂H₅Osi(CH₃)CH₂CH₂CH₂CH₂CH₂CH₂)Si(CH₃)O][SiH(CH₃)O]ₚ₁₀[Si(CH ₃)₂O]_{q2}Si(CH₃)₃,
(CH₃)₃SiO[(Si(OCH₃)₃CH₂CH₂CH₂CH₂CH₂CH₂)Si(CH₃)O][SiH(CH₃)O]ₚ₁₁[Si(CH₃)₂ O]_{q3}Si(CH₃)₃,
(CH₃)₃SiOSi(OC₂H₅)₂O[SiH(C₂H₅)O]ₚ₁₂Si(CH₃)₃,
(CH₃)₃SiO[(Si(OC₂H₅)₂CH₂CH₂CH₂CH₂CH₂CH₂)Si(C₂H₅)]O[SiH(C₂H₅)O]ₚ₁₃Si(CH₃) ₃,
(CH₃)₃SiO[(C₂H₅OSi(CH₃)CH₂CH₂CH₂CH₂CH₂CH₂)Si(C₂H₅)]O[SiH(C₂H₅)O]ₚ₁₄Si(C H₃)₃,
C₂H₅OSi(CH₃)₂CH₂CH₂CH₂CH₂CH₂CH₂(CH₃)₂SiO[HSi(CH₃)₂OSiC₆H₅O]ₚ₁₅Si(CH₃)₂ H,
Si(OCH₃)₃CH₂CH₂CH₂CH₂CH₂CH₂(CH₃)₂SiO[HSi(CH₃)₂OSiC₆H₅O]ₚ₁₆Si(CH₃)₂H,
H(CH₃)₂SiO[(C₂H₅OSi(CH₃)₂CH₂CH₂CH₂)Si(CH₃)O][HSiCH₃O]ₚ₁₇Si(CH₃)₂H,
H(CH₃)₂SiO[(C₂H₅OSi(CH₃)₂CH₂CH₂CH₂CH₂)Si(CH₃)O][HSiCH₃O]ₚ₁₈Si(CH₃)₂H,
H(CH₃)₂SiO[(C₂H₅OSi(CH₃)₂CH₂CH₂CH₂CH₂CH₂CH₂)Si(CH₃)O][HSiCH₃O]ₚ₁₉Si(C H₃)₂H,
H(CH₃)₂SiO[(C₂H₅OSi(CH₃)₂CH₂CH₂CH₂CH₂CH₂CH₂CH₂CH₂)Si(CH₃)O][HSiCH₃O ]ₚ₂₀Si(CH₃)₂H,
H(CH₃)₂SiO[(C₂H₅OSi(CH₃)₂CH₂CH₂CH₂CH₂CH₂CH₂CH₂CH₂CH₂CH₂)Si(CH₃)O][H SiCH₃O]ₚ₂₁Si(CH₃)₂H,
H(CH₃)₂SiO[(Si(OCH₃)₃CH₂CH₂C₆H₄CH₂CH₂)Si(CH₃)O][HSiCH₃O]ₚ₂₂Si(CH₃)₂H,
H(CH₃)₂SiO[(Si(OCH₃)₃CH₂C₆H₄CH₂CH₂CH₂)Si(CH₃)O][HSiCH₃O]ₚ₂₃Si(CH₃)₂H,
H(CH₃)₂SiO[(Si(OCH₃)₃CH₂C₆H₄CH₂CH₂)Si(CH₃)O][HSiCH₃O]ₚ₂₄Si(CH₃)₂H,
H(CH₃)₂SiO[(Si(OCH₃)₃C₆H₄CH₂CH₂)Si(CH₃)O][HSiCH₃O]ₚ₂₅Si(CH₃)₂H,
H(CH₃)₂SiO[(Si(OCH₃)₃CH₂CH₂CH₂)Si(CH₃)O][HSiCH₃O]ₚ₂₆Si(CH₃)₂H,
H(CH₃)₂SiO[(Si(OCH₃)₃CH₂CH₂CH₂CH₂)Si(CH₃)O][HSiCH₃O]ₚ₂₇Si(CH₃)₂H,
H(CH₃)₂SiO[(Si(OCH₃)₃CH₂CH₂CH₂CH₂CH₂CH₂)Si(CH₃)O][HSiCH₃O]ₚ₂₈Si(CH₃)₂H ,
H(CH₃)₂SiO[(Si(OCH₃)₃CH₂CH₂CH₂CH₂CH₂CH₂CH₂CH₂)Si(CH₃)O][HSiCH₃O]ₚ₂₉Si (CH₃)₂H,
H(CH₃)₂SiO[(Si(OCH₃)₃CH₂CH₂CH₂CH₂CH₂CH₂CH₂CH₂CH₂CH₂)Si(CH₃)O][HSiC H₃O]ₚ₃₀Si(CH₃)₂H,
H(CH₃)₂SiO[(Si(OCH₃)₃CH₂CH₂C₆H₄CH₂CH₂)Si(CH₃)O][HSiCH₃O]ₚ₃₁Si(CH₃)₂H,
H(CH₃)₂SiO[(Si(OCH₃)₃CH₂C₆H₄CH₂CH₂CH₂)Si(CH₃)O][HSiCH₃O]ₚ₃₂Si(CH₃)₂H,
H(CH₃)₂SiO[(Si(OCH₃)₃CH₂C₆H₄CH₂CH₂)Si(CH₃)O][HSiCH₃O]ₚ₃₃Si(CH₃)₂H,
H(CH₃)₂SiO[(Si(OCH₃)₃C₆H₄CH₂CH₂)Si(CH₃)O][HSiCH₃O]ₚ₃₄Si(CH₃)₂H,
H(CH₃)₂SiO[(Si(OCH₃)₃CH₂CH₂C₆H₄CH₂CH₂)Si(CH₃)O][HSiCH₃O]ₚ₃₅Si(CH₃)₂H,
H(CH₃)₂SiO[(CH₃O)Si(CH₃)CH₂CH₂CH₂CH₂CH₂CH₂Si(CH₃)₂OSiC₆H₅O]ₚ₃₆[HSi(CH ₃)₂OSiC₆H₅O]_{q4}Si(CH₃)₂H,
H(CH₃)₂SiO[Si(OCH₃)₂CH₂CH₂CH₂CH₂CH₂CH₂Si(CH₃)₂OSiC₆H₅O]ₚ₃₇[HSi(CH₃)₂O SiC₆H₅O]_{q5}Si(CH₃)₂H,
C₂H₅O(CH₃)₂SiO[SiH(CH₃)O]ₚ₃₈[SiCH₃(C₆H₅)O]_{q6}Si(CH₃)₂H,
Si(OC₂H₅)₃CH₂CH₂CH₂CH₂CH₂CH₂(CH₃)₂SiO[SiH(CH₃)O]ₚ₃₉[SiCH₃(C₆H₅)O]_{q7}Si(C H₃)₂H,
C₂H₅OSi(CH₃)₂CH₂CH₂CH₂CH₂CH₂CH₂(CH₃)₂SiO[SiH(CH₃)O]ₚ₄₀[SiCH₃(C₆H₅)O]_{q8} Si(CH₃)₂H,
H(CH₃)₂SiO(C₂H₅O)Si(CH₃)O[SiH(CH₃)O]ₚ₄₁[SiCH₃(C₆H₅)O]_{q9}Si(CH₃)₂H and
H(CH₃)₂SiO[Si(OC₂H₅)₃CH₂CH₂CH₂Si(CH₃)]O[SiH(CH₃)O]ₚ₄₂[SiCH₃(C₆H₅)O]_{q10}Si( CH₃)₂H
are optionally used. In these groups, p1 to p42 and q1 to q10 are number of 1 to 100. The alkoxysilyl compound having the hydrosilyl group preferably has the hydrosilyl group of 1 to 99 in a monomolecular thereof.

As a silane-coupling agent having an alkoxy group without an amino group, an alkoxysilyl compound having a hydrosilyl group can be exemplified. For example,
(C₂H₅O)₃SiCH₂CH=CH₂,
(CH₃O)₃SiCH₂CH₂CH=CH₂,
(C₂H₅O)₃SiCH₂CH₂CH=CH₂,
(CH₃O)₃SiCH₂CH₂CH₂CH₂CH=CH₂,
(C₂H₅O)₃SiCH₂CH₂CH₂CH₂CH=CH₂,
(C₂H₅O)3SiCH₂CH₂CH₂CH₂CH₂CH₂CH=CH₂,
(CH₃O)₃SiCH₂(CH₂)₇CH=CH₂,
(C₂H₅O)₂Si(CH=CH₂)OSi(OC₂H₅)CH=CH₂,
(CH₃O)₃SiCH₂CH₂C₆H₄CH=CH₂,
(CH₃O)₂Si(CH=CH₂)O[SiOCH₃(CH=CH₂)O]ₜ₁Si(OCH₃)₂CH=CH₂,
(C₂H₅O)₂Si(CH=CH₂)O[SiOC₂H₅(CH=CH₂)O]ₜ₂Si(OC₂H₅)₃,
(C₂H₅O)₃SiCH₂CH₂CH₂Si(CH₃)₂OSi(CH₃)₂CH₂CH₂[Si(CH₃)₂O]ₜ₃CH=CH₂,
(CH₃O)₃SiCH₂CH₂CH₂Si(CH₃)₂OSi(CH₃)₂CH₂CH₂[Si(CH₃)₂O]ₜ₄CH=CH₂,
CH₃O(CH₃)₂SiCH₂CH₂CH₂Si(CH₃)₂OSi(CH₃)₂CH₂CH₂[Si(CH₃)₂O]ₜ₅CH=CH₂,
(C₂H₅O)₂CH₃SiCH₂CH₂CH₂Si(CH₃)₂OSi(CH₃)₂CH₂CH₂[Si(CH₃)₂O]ₜ₆CH=CH,
(C₂H₅O)₃SiCH₂CH₂CH₂Si(CH₃)₂OSi(CH₃)₂CH₂CH₂[Si(CH₃)₂O]ₜ₇CH=CH,
(C₂H₅O)₃SiCH₂CH₂CH₂Si(CH₃)₂OSi(CH₃)₂CH₂CH₂(Si(CH₃)₃O)Si(CH₃)O[SiCH₃(-)O] ᵤ₁Si(CH₃)₃CH=CH₂,
(C₂H₅O)₃SiCH₂CH₂CH₂Si(CH₃)₂OSi(CH₃)₂CH₂CH₂(Si(CH₃)₃O)Si(CH₃)O[SiCH₃(-)O] ᵤ₂[Si(CH₃)₂O]ₜ8Si(CH₃)₃CH=CH₂,
(C₂H₅O)₂Si(CH=CH₂)O[SiCH₃(OC₂H₅)O]ᵤ₃Si(OC₂H₅)₂CH=CH₂,
(C₂H₅O)₂Si(CH=CH₂)O[Si(OC₂H₅)₂O]ᵤ₄Si(OC₂H₅)₂CH=CH₂ and
(C₂H₅O)₂Si(CH=CH₂)O[Si(OC₂H₅)₂O]ᵤ₅Si(OC₂H₅)₂CH=CH₂
are optionally used. In these groups, t1 to t8 and u1 to u5 are number of 1 to 30. The alkoxysilyl compound having the hydrosilyl group has preferably the vinyl group of 1 to 30 in the monomolecular thereof.

The reaction of these vinyl groups and SiH groups may be accelerated by the metal catalyst, e.g. a compound including platinum and thus, the substrate sheets and rubber sheets may be joined.

As a silane-coupling agent having an alkoxy group without an amino group, an alkoxysilyl compound having the alkoxysilyl group at both terminals may be exemplified. For example,
(C₂H₅O)₃SiCH₂CH₂Si(OC₂H₅)₃,
(C₂H₅O)₂CH₃SiCH₂CH₂Si(OC₂H₅)₃,
(C₂H₅O)₃SiCH=CHSi(OC₂H₅)₃,
(CH₃O)₃SiCH₂CH₂Si(OCH₃)₃(CH₃O)₃SiCH₂CH₂C₆H₄CH₂CH₂Si(OCH₃)₃,
(CH₃O)₃Si[CH₂CH₂]₃Si(OCH₃)₃,
(CH₃O)₂Si[CH₂CH₂]₄Si(OCH₃)₃,
(C₂H₅O)₂Si(OC₂H₃)₂,
(CH₃O)₂CH₃SiCH₂CH₂Si(OCH₃)₂CH₃,
(C₂H₅O)₂CH₃SiOSi(OC₂H₅)₂CH₃,
(CH₃O)₃SiO[Si(OCH₃)₂O]ᵥ₁Si(OCH₃)₃,
(C₂H₅O)₃SiO[Si(OC₂H₅)₂O]ᵥ₂Si(OC₂H₅)₃ and
(C₃H₇O)₃SiO[Si(OC₃H₇)₂O]ᵥ₃Si(OC₃H₇)₃
are optionally used. In these groups, v1 to v3 are number of 0 to 30.

As a silane-coupling agent having an alkoxy group without an amino group, an alkoxysilyl compound having hydrolytic group-containing silyl group can be exemplified. For example, an easily-hydrolytic organosilane is optionally used. Particularly,
CH₃Si(OCOCH₃)₃, (CH₃)₂Si(OCOCH₃)₂, n-C₃H₇Si(OCOCH₃)₃. CH₂=CHCH₂Si(OCOCH₃)₃, C₆H₅Si(OCOCH₃)₃, CF₃CF₂CH₂CH₂Si(OCOCH₃)₃, CH₂=CHCH₂Si(OCOCH₃)₃, CH₃OSi(OCOCH₃)₃, C₂H₅OSi(OCOCH₃)₃, CH₃Si(OCOC₃H₇)₃, CH₃Si[OC(CH₃)=CH₂]₃, (CH₃)₂Si[OC(CH₃)=CH₂]₃, n-C₃H₇Si[OC(CH₃)=CH₂]₃, CH₂=CHCH₂Si[OC(CH₃)=CH₂]₃, C₆H₅Si[OC(CH₃)=CH₂]₃, CF₃CF₂CH₂CH₂Si[OC(CH₃)=CH₂]₃, CH₂=CHCH₂Si[OC(CH₃)=CH₂]₃, CH₃OSi[OC(CH₃)=CH₂]₃, C₂H₅OSi[OC(CH₃)=CH₂]₃, CH₃Si[ON=C(CH₃)C₂H₅]₃, (CH₃)₂Si[ON=C(CH₃)C₂H₅]₂, n-C₃H₇Si[ON=C(CH₃)C₂H₅]₃, CH₂=CHCH₂Si[ON=C(CH₃)C₂H₅]₃, C₆H₅Si[ON=C(CH₃)C₂H₅]₃, CF₃CF₂CH₂CH₂Si[ON-C(CH₃)C₂H₅]₃, CH₂=CHCH₂Si[ON=C(CH₃)C₂H_{5]3}, CH₃OSi[ON=C(CH₃)C₂H₅]₃, C₂H₅OSi[ON=C(CH₃)C₂H₅]]₃, CH₃Si[ON=C(CH₃)C₂H₅]₃, CH₃Si[N(CH₃)]₃, (CH₃)₂Si[N(CH₃)]₂, n-C₃H₇Si[N(CH₃)]₃, CH₂=CHCH₂Si[N(CH₃)]₃, C₆H₅Si[N(CH₃)]₃, CF₃CF₂CH₂CH₂Si[N(CH₃)]₃, CH₂=CHCH₂Si[N(CH₃)]₃, CH₃OSi[N(CH₃)]₃, C₂H₅OSi[N(CH₃)]₃ and CH₃Si[N(CH₃)]₃ are included.

As a silane-coupling agent containing an amino group and having alkoxy group, an available silane-coupling agent is included. Particularly, an alkoxysilyl compound containing the amino group exemplified by N-2-(aminoethyl)-3-aminopropylmethyldimethoxysilane (KBM-602), N-2-(aminoethyl)-3-aminopropyltrimethoxysilane (KBM-603), N-2-(aminoethyl)-3-aminopropyltriethoxysilane (KBE-603), 3-aminopropyltrimethoxysilane (KBM-903), 3-aminopropyltriethoxysilane (KBE-903), 3-triethoxysilyl-N-(1,3-dimethyl-butylidene) propylamine (KBE-9103), N-phenyl-3-aminopropyltrimethoxysilane (KBM-573) and N-(vinylbenzyl)-2-aminoethyl-3-aminopropyltrimethoxysilane hydrochloride (KBM-575) (all of which is manufactured by Shin-Etsu Chemical Co., Ltd.; trade names) may be used. Further, an alkoxysilyl compound containing an amino group exemplified by 3-aminopropyltrimethoxysilane (Z-6610), 3-aminopropyltrimethoxysilane (Z-6611), 3-(2-aminoethyl)aminopropyltrimethoxysilane (Z-6094), 3-phenylaminopropyltrimethoxysilane (Z-6883), and N[3-(trimethoxysilyl)propyl]-N'-[(ethenylphenyl)methyl]-1,2-ethanediamine hydrochloride (Z-6032) (all of which is manufactured by Dow Corning Toray Co., Ltd.; trade names) may be used.

When the protecting-substrate sheets are made from the metal, ceramics or glass and the outermost flow-path-retaining substrate sheets 10, 50 are made from the silicone rubber, these sheets are preferably joined through the direct ether bond. In this case, the active groups such as the hydroxy group are generated on the faces of the protecting-substrate sheets and the flow-path-retaining substrate sheets 10, 50 by the corona treatment, plasma treatment or ultraviolet irradiation treatment (the usual UV treatment and excimer UV treatment). Thus, the ether bond is formed between the protecting-substrate sheets and the flow-path-retaining substrate sheets 10, 50 through the dehydration thereof by compression bond under pressurization or reduced pressure.

When the protecting-substrate sheets are made from the metal, ceramics or glass and the flow-path-retaining substrate sheets 10, 50 are made from the silicone rubber containing the non-silicone rubber, these sheets may be joined by a covalent bond of an oxygen-carbon bond, carbon-carbon bond, and oxygen-silicon bond through the silane-coupling agent having the alkoxy group without the amino group. In this case, the active groups such as the hydroxy group are generated on the faces of the protecting-substrate sheets and the flow-path-retaining substrate sheets 10, 50 by the corona treatment, plasma treatment or ultraviolet irradiation treatment (the usual UV treatment and excimer UV treatment). Thus, the covalent bonds are formed by the bond under normal atmospheric pressure, the pressurization or reduced pressure at normal temperature or heating temperature by applying the silane-coupling agent including the alkoxy group or alkoxy-equivalent group, and optionally an unsaturated group, epoxy group, ureido group, sulfide group or isocyanate group without the amino group.

When the protecting-substrate sheets are made from the resin and the flow-path-retaining substrate sheets 10, 50 are made from the silicone rubber containing the non-silicone rubber, these sheets may be joined by chemical bonds of the covalent bond of the oxygen-silicon bond through the silane-coupling agent having the amino group and alkoxy group, and the hydrogen bond of the hydroxy group-amino group. Alternatively, these sheets are may be joined by a covalent bond such as an amide bond or imino bond by a carboxyl group or carbonyl group which is newly produced. In this case, the active groups such as the hydroxy group arc generated on the faces of the protecting-substrate sheets and the flow-path-retaining substrate sheets 10, 50 by the corona treatment, plasma treatment or ultraviolet irradiation treatment (the usual UV treatment and excimer UV treatment). Thus, since the silane-coupling agent including the alkoxy group or an alkoxy-equivalent group and the amino group is applied to these sheets, the chemical bonds are produced when these sheets are compressed under the normal atmospheric pressure, pressurization or reduced pressure at the normal temperature or heating temperature. In this instance, the amino group of the silane-coupling agent is easily adsorbed to the resin. When the resin is the polycarbonate resin,cycloolefin resin, polyethylene terephthalate resin, acryl resin or epoxy resin, these sheets are rapidly, strongly and easily bonded by being especially progressed the reaction. Among these resins, when the resin is the polycarbonate resin or the polycarbonate resin, superior water resistance is exhibited.

Approach of the active groups such as the hydroxy group of the protecting-substrate sheets and the flow-path-retaining substrate sheets 10, 50 or a reactive functional group of the silane-coupling agent, which reacts therewith, is accelerated by removing gaseous media of contact boundaries under reduced pressure or vacuum conditions. As the reduced pressure or vacuum conditions, for example, 50 torr or less, more particularly, the reduced pressure conditions of 50 to 10 torr or the vacuum conditions of less than 10 torr, more particularly, less than 10 to 1x10⁻³ torr, preferably less than 10 to 1x10⁻² torr. Alternatively, the approach thereof may be accelerated by adding a stress (a load) e.g. 10 to 200 kgf to the contact boundaries thereof, and further by heating the contact boundaries thereof. The whole surfaces of the bonded faces of the substrate sheet 10, 50 and the protecting-substrate sheets are preferably homogeneously pressurized. If the values fall outside the above range, the pressure could not homogeneously be applied.

One embodiment of the three-dimensional microchemical chip 1 shown in Fig. 1 is produced as follows. A large size sheet for the silicone rubber-made flow-path-supporting substrate sheet and the silicone rubber-made flow-path-retaining substrate sheet is made from a composite containing the thermally conductive filler powder and a silicone rubber raw material component. Alternatively, a large size sheet for the flow-path-retaining substrate sheets is made from a resin raw material composite such as a polyimine raw material component. The flow-path-retaining substrate sheets 10, 30, 50 and the flow-path-supporting substrate sheets 20, 40 are cut in rectangles from the large size sheet. As shown in Fig. 1, the fluid-sample-injecting holes 11, the fluid-sample-receiving holes 21, 31, 41, the micro flow path 22, 42, the fluid-sample-delivering holes 23 and the fluid-sample-draining holes 13 are defined by piercing the flow-path-retaining substrate sheets 10, 30, 50 and the flow-path-supporting substrate sheets 20, 40. The piercing of these sheets is carried out by using a laser processing, drill processing or punch processing.

The flow-path-retaining substrate sheets 10, 30, 50 and the flow-path-supporting substrate sheets 20, 40 are washed with alcohol and water. The second face 14b of the flow-path-retaining substrate sheet 10, both of the first faces 24a, 34a, 44a and the second faces 24b, 34b, 44b of the flow-path-supporting substrate sheet 20, 40 and the flow-path-retaining substrate sheet 30, and the first face 54a of the flow-path-retaining substrate sheet 50 are subjected to the corona discharge treatment. Thereby the hydroxy groups are newly generated on these faces. The flow-path-retaining substrate sheets 10, 30, 50 and the flow-path-supporting substrate sheets 20, 40 are stacked under the conditions of the normal atmospheric pressure. In this case, the pressure may be optionally reduced under 10 torr or less. These sheets are pressed by thermal compression e.g. at 80 to 120°C while optionally pressing at 10 to 200 kgf. In the result, the ether bond is generated by the dehydration between the hydroxy groups on the flow-path-retaining substrate sheets 10, 30, 50 and the flow-path-supporting substrate sheets 20, 40 which are faced each other and thus, these sheets are joined. In this way, the three-dimensional microchemical chip 1 can be obtained.

Incidentally, although the embodiment in which the corona discharge treatment is conducted to the flow-path-retaining substrate sheets 10, 30, 50 and the flow-path-supporting substrate sheets 20, 40 is mentioned above, the atmospheric pressure plasma treatment or ultraviolet irradiation treatment (the usual UV treatment and excimer UV treatment) may be conducted thereto. By these treatments, the active groups of the hydroxy groups are generated on the surfaces of the organic flow-path-retaining substrate sheets 10, 30, 50 and the flow-path-supporting substrate sheets 20, 40. Further, the active groups exemplified by the carboxyl group or carbonyl group are generated thereon.

The flow-path-retaining substrate sheets 10, 30, 50 and the flow-path-supporting substrate sheets 20, 40 originally have the hydroxy group or do not originally have the hydroxy group depending on the composition of the raw materials. When these sheets do not have the hydroxy group on the surfaces of them, the hydroxy group is effectively generated thereon by the corona treatment, atmospheric pressure plasma treatment or ultraviolet irradiation treatment (the usual UV treatment and excimer UV treatment).

The optimal treatment conditions vary according to the history and kinds of the materials of the flow-path-retaining substrate sheets 10, 30, 50 and the flow-path-supporting substrate sheets 20, 40. It is important to conduct the treatment of the surface thereof until obtaining a surface tension up to 55 kJ/m continuously. A sufficient adhesive strength can be obtained thereby.

Particularly, the corona discharge treatment for the flow-path-retaining substrate sheets 10, 30, 50 and the flow-path-supporting substrate sheets 20, 40 is conducted under the conditions of e.g. power source: AC100V, output voltage: 0 to 20kV, oscillating frequency: 0 to 40kHz for 0.1 to 60 seconds, and temperature: 0 to 60°C by using an apparatus for corona surface modification (e.g. trade name of CoronaMaster manufactured by Shinko Electric & Instrumentation Co., Ltd.).

The atmospheric pressure plasma treatment for the flow-path-retaining substrate sheets 10, 30, 50 and the flow-path-supporting substrate sheets 20, 40 is conducted under conditions of e.g. plasma processing speed: 10 to 100mm/s, power source: 200 or 220V AC (30A), compressed air: 0.5 MPa (1 NL/min.), and 10 kHz/300W to 5 GHz. electric power: 100 to 400W, and irradiation period of time: 1 to 60 seconds by using an air plasma generator (e.g. trade name of Aiplasma manufactured by Matsushita Electric Industrial Co., Ltd.).

The ultraviolet irradiation treatment for the flow-path-retaining substrate sheets 10, 30, 50 and the flow-path-supporting substrate sheets 20, 40 is conducted under conditions of e.g. wave length: 200 to 400nm, power source: 100V AC, light source peak illuminance: 400 to 3000 mW/cm², irradiation period of time: 0.1 to 60 seconds by using an ultraviolet-light emitting diode (UV-LED) irradiator (e.g. UV irradiator: trade name of ZUV-C30H manufactured by OMRON Corporation).

Incidentally, when the flow-path-retaining substrate sheets 10, 30, 50 and the flow-path-supporting substrate sheets 20, 40 are joined, both of the first and the second faces of these sheets, which are faced each other, may be dipped into the silane-coupling agent of the molecular adhesive, followed by contacting to the protecting-substrate sheets (not shown). As substituted for dipping into the silane-coupling agent, it may be sprayed onto the faces of these sheets. The period of time of dipping or spraying is not restricted, it is important to homogeneously wet the substrate surfaces of the protecting-substrate sheets.

The protecting-substrate sheets, which are applied the silane-coupling agent, are dried by putting in an oven, by blasting the air using a dryer or by irradiating high frequency wave while heating. The heating and drying are conducted at a temperature range of 50 to 250°C for 1 to 60 minutes. If the temperature is less than 50°C, the reaction between the hydroxy group generated on the surfaces of the protecting-substrate sheets and the silane-coupling agent takes a long time, decreasing in productivity, and increasing in cost. If the temperature is more than 250°C, the surfaces of the protecting-substrate sheets are deformed or degraded even for short period of time. If the time of heating and drying is less than 1 minute, thermal conductivity is insufficient and thus, the hydroxy group of the surfaces of the protecting-substrate sheets and the silane-coupling agent are insufficiently bonded. If the time thereof is more than 60 minute, the productivity decreases.

When the reaction between the hydroxy group of the surfaces of the protecting-substrate sheets and the silane-coupling agent is insufficient, the dipping and drying may be repeated about 1 to 5 times. Therefore, the time of the dipping and drying of each time can decrease and thus, the reaction can be sufficiently progressed by increasing the reaction frequency.

According to explanation with reference to Fig. 1, as an embodiment in the case of microsynthesis, the three-dimensional microchemical chip 1 is used as follows. The three-dimensional microchemical chip 1 is installed to an apparatus body of a microreactor (not shown). Syringes (not shown) are air-tightly inserted in the fluid-sample-injecting holes 11a, 11b of the flow-path-retaining substrate sheet 10 for covering. And the fluid samples of the liquid specimen and liquid reagent are imported from the syringes into the micro flow paths 22a, 22b through the fluid-sample-receiving holes 21a, 21b while pressurizing at a pressure between more than 100 kPa to 3 MPa or less, respectively. Both of the fluid samples are joined by flowing in the micro flow path 22b through the fluid-sample-receiving holes 21b, followed by being mixed and mutually reacted. The fluid samples, which are flowed in the micro flow path 22d thereafter, are separated from a main channel. Thus, the fluid samples are flowed into the micro flow paths 22a, 22g.

A syringe is air-tightly inserted in the fluid-sample-injecting holes 11e of the fluid-sample-retaining substrate sheet 10 on the micro flow path 22e side. The fluid sample of the liquid reagent is imported from the syringe into the fluid-sample-receiving hole 21e through the fluid-sample-injecting holes 11e while pressurizing in the same manner as above. The fluid sample is flowed in the micro flow path 22f, followed by being joined, mixed and mutually reacted. The fluid sample is drained from the fluid-sample-draining hole 13a through the fluid-sample-delivering hole 23a. The drained fluid sample includes a desired fluid sample containing a specific product which is infinitesimally synthesized or a product and by-product which are generated by chemical reaction.

In addition, a syringe is air-tightly inserted in the fluid-sample-injecting holes 11g of the fluid-sample-retaining substrate sheet 10 on the micro flow path 22g side. The fluid sample of the liquid reagent is imported from the syringe into the micro flow path 42h through the fluid-sample-injecting holes 21g while pressurizing in the same manner as above. The fluid sample is flowed in the micro flow path 42h, followed by being joined, mixed and mutually reacted. The fluid sample is drained from the fluid-sample-draining hole 13b through the micro flow path 42h, the fluid-sample-receiving holes 41 i, 31 i, 21 i, the micro flow path 22i and the fluid-sample-delivering hole 23b. The drained fluid sample includes a desired fluid sample containing a specific product which is infinitesimally synthesized or a product and by-product which are generated by chemical reaction.

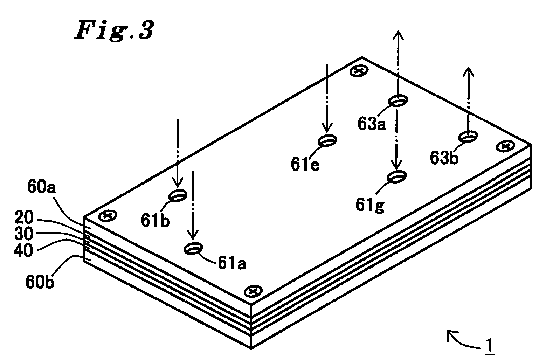

Another embodiment of a three-dimensional microchemical chip 1 is shown in Fig. 3. The three-dimensional microchemical chip 1 has the flow-path-supporting substrate sheets 20, 40 and the flow-path-retaining substrate sheet 30 shown in Fig. 1. The flow-path-supporting substrate sheets 20, 40 and the flow-path-retaining substrate sheet 30 are sandwiched between holders 60a, 60b of two resin plates or metal plates. The holders 60a, 60b have rigidity and inflexibility, and double as protecting substrates. The holders 60a, 60b substitute for the flow-path-retaining substrate sheets 10, 50 shown in Fig. 1. The holders 60a, 60b are subjected to the corona treatment, plasma treatment or ultraviolet irradiation treatment (the usual UV treatment and excimer UV treatment). Thereby the holders 60a, 60b are directly joined to the flow-path-supporting substrate sheets 20, 40, and integrated with the flow-path-supporting substrate sheets 20, 40 and the flow-path-retaining substrate sheet 30. Injection leading holes 61a, 61b, 61e, 61g and drain leading holes 63a, 63b are opened into the holder 60a at positions corresponding to the fluid-sample-injecting holes 11 a, 11b, 11e, 11g and the fluid-sample-draining holes 13a, 13b of the flow-path-retaining substrate sheet 10. The three-dimensional microchemical chip 1 is used to flow the fluid sample into the micro flow paths 22, 42 by pressurizing in the same manner as Fig. 1. The flow-path-supporting substrate sheets 20, 40 and the flow-path-retaining substrate sheet 30 are pressed by the holders 60a, 60b at pressure which is possible to flow the fluid sample into the micro flow paths 22, 42. Thereby the flow-path-supporting substrate sheets 20, 40 and the flow-path-retaining substrate sheet 30 are supported by the holders 60a, 60b so as to not bend.

In the three-dimensional microchemical chip 1 shown in Figs. 1 to 3, a heater (not shown) may be inserted and joined between the flow-path-supporting substrate sheets 20, 40 and the flow-path-retaining substrate sheets 10, 30, 50. Alternatively the heater (not shown) may be placed on the holders or under the holders shown in Fig. 3. A sensor such as an electrode etc. for detecting the specimen, reagent and/or reaction product may be wired in any one of the fluid-sample-injecting holes 11, the fluid-sample-receiving holes 21, 31, 41, the micro flow paths 22, 42, the fluid-sample-delivering holes 23 and the fluid-sample-draining holes 13.

### Embodiments

Embodiments of producing of a three-dimensional microchemical chip 1 of the present invention will be described below.

### (Example 1)

50 parts by weight of methylvinylsilicone rubber as silicone rubber (manufactured by Dow Corning Toray Co., Ltd., trade name: SH1005), 50 parts by weight of polydimethylsiloxane as silicone oil (manufactured by Dow Corning Toray Co., Ltd., trade name: SH200 100cs), 50 parts by weight of magnesium oxide as thermally conductive filler (manufactured by Kyowa Chemical Industry Co., Ltd., trade name: PYROKISUMA (registered trademark) 5301, average particle size: 2µm), 200 parts by weight of magnesium oxide (manufactured by Kyowa Chemical Industry Co., Ltd., trade name: PYROKISUMA (registered trademark) 3320, average particle size: 20µm), 50 parts by weight of aluminum hydroxide Al(OH)₃ (manufactured by Showa Denko K.K., trade name: HIGILITE (registered trademark) H32), 10 parts by weight of calcium oxide CaO (manufactured by Inoue Calcium Corporation, trade name: VESTA PP) as an additive agent and 0.01 parts by weight of a platinum carbonyl cyclovinylmethylsiloxane complex including a vinylmethylcyclosiloxane solution of a platinum complex as a platinum catalyst (manufactured by Gelest, Inc.) were kneaded. A composite for a silicone rubber-made thermally conductive sheet was obtained. The composite was heated and pressurized, and the silicone rubber-made thermally conductive sheet for flow-path-retaining substrate sheets 10, 30, 50 was produced.

100 parts by weight of methylvinylsilicone rubber as a silicone rubber (manufactured by Dow Corning Toray Co., Ltd., trade name: SH851), 0.5 parts by weight of 2,5-dimethyl-2,5-di(tert-butylperoxy)hexane as a peroxide type crosslinking agent (manufactured by Dow Corning Toray Co., Ltd., trade name: PC-4, 50% silica solution) were kneaded. A composite for an intermediate layer was obtained. The composite was heated and pressurized, and a silicone rubber-made thermally conductive sheet for flow-path-supporting substrate sheets 20, 40 was produced.

As shown in Figs. 1 to 4, the flow-path-retaining substrate sheets 10, 30, 50 and the flow-path-supporting substrate sheets 20, 40 were formed and adhered, and a three-dimensional microchemical chip of the present invention through the rubber was obtained.

A three-dimensional microchemical chip which was produced by the above mentioned thermally conductive sheet adhering to 30 shown in Fig. 1 was compared with a three-dimensional microchemical chip which was produced by adhering sheets made from the available SH851U mentioned above. In a result, in order to conduct 30 cycles of PCR, the latter microchemical chip spent a period of time approximately 150% than former.

The three-dimensional microchemical chip 1 of the present invention which was obtained by the above manner was used to amplify deoxyribonucleic acid (DNA). In order to amplify DNA, in a first step (94 to 96°C), target double-stranded DNA was thermally degenerated to single-stranded DNA. In a second step (55 to 60°C), a primer was annealed to the single-stranded DNA. In a third step (72 to 74°C), an elongation reaction was progressed. In this way, the polymerase chain reaction (PCR) was repeated. Heating was conducted by using a thermocouple, Peltier device or infrared ray irradiation. Since the three-dimensional microchemical chip 1 has the silicone rubber-made thermal conductive sheets, the temperature was smoothly risen and reduced and thus, PCR could be progressed uneventfully.

As a comparative example, a three-dimensional microchemical chip, which was excepted from applying of the present invention, was produced by employing a rubber sheet not containing the thermally conductive filler instead of the silicone rubber-made thermally conductive sheet. When the three-dimensional microchemical chip for the comparative example was used to PCR, PCR could not be progressed uneventfully. In this case, the temperature was not smoothly risen and reduced, and a period of time was so long. The three-dimensional microchemical chip of the comparative example decreased efficiency of PCR.

### (Example 2: Examining of water vapor permeability)

### (1) Preparing of a silicone rubber sheet

100 parts by weight of methylvinylsilicone rubber as silicone rubber (manufactured by Dow Corning Toray Co., Ltd., trade name: SH851U) and 0.5 parts by weight of 2,5-dimethyl-2,5-di(tert-butylperoxy)hexane as a peroxide type crosslinking agent (manufactured by Dow Corning Toray Co., Ltd., trade name: PC-4, 50% silica solution) were kneaded. A composite for a sheet was obtained. The composite was heated and pressurized, and a silicone-made sheet was prepared.

### (2) Preparing of a silicone rubber sheet (blending of diatomaceous earth)

100 parts by weight of a methylvinylsilicone rubber as silicone rubber (manufactured by Dow Corning Toray Co., Ltd., trade name: SH851U), 0.5 parts by weight of 2,5-dimethyl-2,5-di(tert-butylperoxy)hexane as a peroxide type crosslinking agent (manufactured by Dow Corning Toray Co., Ltd., trade name: PC-4, 50% silica solution) and 10, 20 or 30 parts by weight of diatomaceous earth (supplied by TOKYO KOGYO BOYEKI SHOKAI, LTD., trade name: CelTix) were kneaded, respectively. Composites for a sheet were obtained. The composites were heated and pressurized, and silicone-made sheets were prepared.

### (3) Preparing of SEP sheet

100 parts by weight of SEP rubber (silicone modified EPDM) (manufactured by Shin-Etsu Chemical Co., Ltd., trade name: SEP-1411-U or SEP-1421-U) which is prepared to modify silicone rubber with ethylene-propylene rubber, 4 part by weight of dicumyl peroxide as a peroxide type crosslinking agent (manufactured by Shin-Etsu Chemical Co., Ltd., trade name: C-12, approx. 40%) and 0.2 parts by weight of N,N'-(m-phenylene)bismaleimide as a vulcanization accelerator (manufactured by Shin-Etsu Chemical Co., Ltd., trade name: SEP-BM) were kneaded, and a molded product for a sheet was obtained. The molded product was heated and pressurized, and SEP sheet was prepared.

### (4) Preparing of EPDM sheet

140 part by weight of ethylene-propylene rubber (EPDM) (manufactured by Mitsui Chemicals, Inc., trade name: EPT3072), 30 parts by weight of silica (manufactured by TOSOH SILICA CORPORATION, trade name: Nipsil (registered trademark) VM3), 1 part by weight of silica (manufactured by Seiko Chemical Co.,Ltd., trade name: Hi-Cross M) and 2 parts by weight of a peroxide type crosslinking agent (manufactured by NOF CORPORATION, trade name: PERHEXA (registered trademark) 25B) were kneaded. A composite for sheet was obtained. The composite was heated and pressurized, and EPDM sheet was prepared.

### (5) Moisture permeability test

The obtained various rubber sheets were subjected to a moisture permeability test while referring to Japanese Industrial Standards L-1099 A-1 method (Calcium chloride method). The above mentioned various rubber sheets formed into 0.2 mm thickness were processed into 18 mm diameter. 10g of calcium chloride was put in 20 mL containers. The containers were stopped by the various rubber sheets and sealed up thereon by O-rings and ring-shaped caps having a hole. Thereafter, the containers were stored in a constant-temperature-humidity compact chamber (manufactured by ESPEC CORP., trade name: SH-241) at a temperature 40°C and a humidity 90 RH% for 72 hours. By comparing the weight before the test with the weight after the test, amount of the moisture permeation was calculated. The results of the moisture permeability test are shown in Table 1.

**Table 1 Result of Moisture permeability test**

| Rubber sheet | Moisture permeation amount (g) |
|---|---|
| SH851U | 0.223 |
| SH851U containing 10 parts by weight diatomaceous earth | 0.203 |
| SH851U containing 20 parts by weight diatomaceous earth | 0.193 |
| SH851 U containing 30 parts by weight diatomaceous earth | 0.172 |
| SEP1411 | 0.013 |
| SEP1412 | 0.021 |
| EPDM | 0.013 |

As shown in Table 1, it is obvious that the moisture permeability is decreased by adding the diatomaceous earth into the silicone rubber. The decrease of the moisture permeability is proportional to the additive amount of the diatomaceous earth. In addition, it is confirmed that the moisture permeation amounts of SEP sheet and EPDM sheet decrease further.

### (Example 3: Examining of fluid flow properties)

### (1) Producing of a three-dimensional microchemical chip

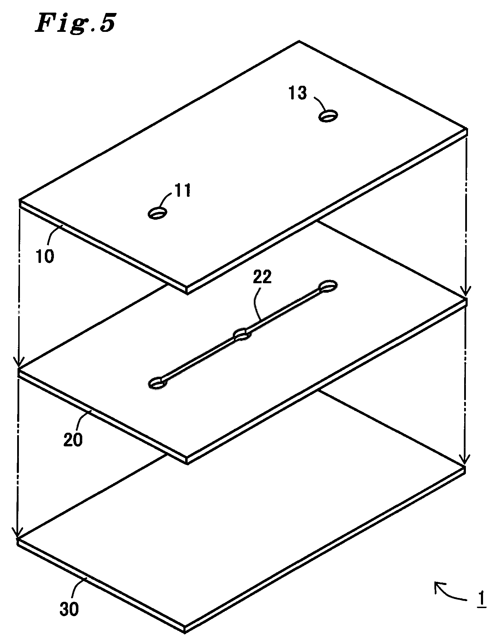

A simply structured three-dimensional microchemical chip consisting of two sheets was produced by employing a polycarbonate plate 10 of a flow-path-retaining substrate sheet and the above various rubber sheets 20 of a flow-path-supporting substrate sheet (see Fig. 5). (However, the sheets 20 were the plate which was processed so as to define a recessing-shaped flow path 22, and the sheet 10 was the flat sheet which was pierced so as to form a fluid-sample-injecting part 11 and draining part 13. A sheet 30 was not used.) The polycarbonate rein plate 10 was made from Panlite (registered trademark) LV-2225Y (manufactured by TEIJIN LIMITED) and had a size of 50x50 mm and 2 mm thickness. The sheet 20 had the flow path 22 corresponding to a central region of the polycarbonate resin plate 10. The flow path 22 had 100 µm width, 30 µm deepness and 40 mm length. In the polycarbonate resin plate 10, the fluid-sample-injecting part 11 and the fluid-sample-draining part 13 having each 2 mm diameter respectively were pierced at positions corresponding to both ends of the flow path 22. The various rubber sheets 20 had the size of the same as the polycarbonate resin plate 10 and 500 µm thickness. The polycarbonate resin plate 10 and the various rubber sheets 20 were joined by using molecular adhesive technology as follows, and microchemical chips 1 were obtained. The molded polycarbonate resin plate 10 was washed with ethanol. A surface thereof was activated by three times of a corona discharge treatment. Conditions of the corona discharge treatment were 1 mm gap length, 13.5 kV voltage and 70 mm/second. Thereafter, the polycarbonate resin plate 10 was dipped into 0.1% by weight of 3-(2-aminoethylamino)propyltrimethoxysilane of a silane-coupling agent in an ethanol solution. The polycarbonate resin plate 10 was dried by using a blow-gun, heated at 80°C for 10 minutes and washed with ethanol again. SH851U and SH851U containing 30 parts by weight of diatomaceous earth as the various rubber sheets 20 were washed with ethanol. Surfaces of both rubber sheets 20 were activated by three times of the corona discharge treatment in the same conditions as above. In addition, both of the various rubber sheets 20 having SEP1411, SEP1412 or EPDM were washed with ethanol and activated by three times of the corona discharge treatment in the same conditions as above. Thereafter, these sheets were dipped into 1.0% by weight of 3-mercaptopropyltrimethoxysilane of the silane-coupling agent in the ethanol solution, dried by using the blow-gun, heated at 80°C for 10 minutes and washed with ethanol again. The polycarbonate resin plate 10 and the treated various rubber sheets 20 were stacked so that the fluid-sample-injecting part 11 and the fluid-sample-draining part 13 were positioned at the both ends of the flow path 22. The resultant products were pressed by thermal compression at 80°C for 10 minutes while pressing 70 kgf. The microchemical chips 1 were obtained.

### (2) Fluid flow evaluation test

When ion-exchange water was dropped into the fluid-sample-injecting part 11 of each the microchemical chips 1 employing the various rubber sheets, flow of the ion-exchange water was evaluated by capillarity phenomenon. The results of the fluid flow evaluation are shown in Table 2.

**Table 2 Fluid flow evaluation**

| Flow-path-supporting substrate sheet of the microchemical chip (Rubber sheet) | Flow distance of the ion-exchange water in 1 min. after dropping (mm) |
|---|---|
| SH851U | 0 |
| SH851U containing 30 parts by weight diatomaceous earth | 10 |
| SEP1411 | 40 (Reached to the fluid-sample-draining part 13) |
| SEP1412 | 40 (Reached to the fluid-sample-draining part 13) |
| EPDM | 40 (Reached to the fluid-sample-draining part 13) |

As shown in Table 2, it is confirmed that the flow rate of the ion-exchange water by the capillarity phenomenon is increased depending on adding the diatomaceous earth into the silicone rubber. Further, in the microchemical chips 1 which employ SEP1411, SEP1142 or EPDM, the ion-exchange water reaches to the fluid-sample-draining part 13.

### (Example 4: Examining of adhesive properties between PC and EPDM in a three-dimensional microchemical chip)

### (1) Preparing of EPDM sheet

140 parts by weight of ethylene-propylene rubber (EPDM) (manufactured by Mitsui Chemicals, Inc., trade name: EPT3072), 30 parts by weight of silica (manufactured by TOSOH SILICA CORPORATION, trade name: Nipsil (registered trademark) VM3), 1 part by weight of silica (manufactured by Seiko Chemical Co.,Ltd., trade name: Hi-Cross M) and 2 parts by weight of a peroxide type crosslinking agent (manufactured by NOF CORPORATION, trade name: PERHEXA (registered trademark) 25B) were kneaded. A composite for sheet was obtained. The composite was heated and pressurized, and EPDM sheet was prepared.

### (2) Producing of a three-dimensional microchemical chip by joining of EPDM sheet and PC (polycarbonate) sheet

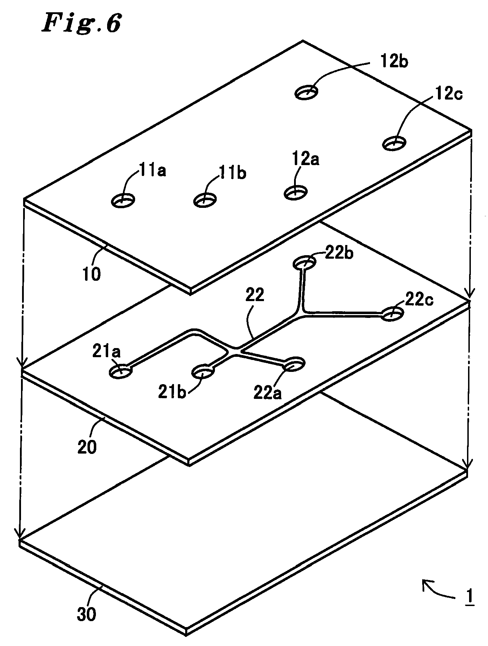

A microchemical chip 1 shown in Fig. 6 was produced by employing polycarbonate plates 10, 30 as the flow-path-retaining substrate sheet and EPDM sheet 20 as the flow-path-supporting substrate sheet. The polycarbonate plates 10, 30 were made from polycarbonate for optics application (manufactured by Takiron Co., Ltd., trade name: PCSM PS610) and had a size of 30x40 mm and 2 mm thickness. The EPDM sheet 20 had the size of the same as the polycarbonate plates 10, 30 and 500 µm thickness. The EPDM sheet 20 was formed by the composition of the same as Example 2 employing EPT3072 (manufactured by Mitsui Chemicals, Inc., trade name). A flow path 22 was defined on the EPDM sheet 20 by using a laser processing apparatus (supplied by COMNET Corporation, trade name: LaserPro SPILIT, conditions for processing: speed 6.0, power 90, PPI 400) as shown in Fig. 6. The flow path 22 included fluid-sample-injecting sites 21 a, 21b and fluid-sample-draining sites 22a, 22b, 22c having each 1 mm diameter. The flow path 22 had a branching channel shape having 500 µm width. The formed EPDM sheet 20 was washed with ethanol. A surface of the EPDM sheet 20 was activated by three times of the corona discharge treatment of which conditions were 1 mm gap length, 13.5 kV voltage and 70 mm/second. Thereafter, the EPDM sheet 20 was dipped into 1.0% by weight of 3-mercaptopropyltrimethoxysilane of the silane-coupling agent in the ethanol solution, dried by using a blow-gun, heated at 80°C for 10 minutes and washed with ethanol again. Fluid-sample-injecting holes 11a, 11b and fluid-sample-draining holes 12a, 12b, 12c were drilled into the polycarbonate plate 10 for covering. The polycarbonate plate 10 for covering and the polycarbonate plate 30 for bottom face supporting were washed with ethanol. Surfaces of the polycarbonate plates 10, 30 were activated by three times of the corona discharge treatment in the same conditions as above. The polycarbonate plates 10, 30 were dipped into 1.0% by weight of 3-mercaptopropyltrimethoxysilane of the silane-coupling agent in the ethanol solution, dried by using the blow-gun, heated at 80°C for 10 minutes and washed with ethanol again. The EPDM sheet 20 was sandwiched between the polycarbonate plates 10, 30 so that the fluid-sample-injecting sites 21a, 21b and the fluid-sample-draining sites 22a, 22b, 22c were corresponded to the fluid-sample-injecting holes 11 a, 11b and the fluid-sample-draining holes 12a, 12b, 12c, respectively. The resultant products were pressed by thermal compression at 80°C for 10 minutes while pressing 70 kgf. The microchemical chip 1 was obtained.

### (3) Pressure resistance test

The fluid-sample-injecting hole 11b and the fluid-sample-draining holes 12a, 12b, 12c were closed, and pressurized air was introduced into the fluid-sample-site 21 a through the fluid-sample-injecting hole 11a. In the result, the microchemical chip 1 exhibited pressure resistance up to 1.5 MPa.

### (Example 5: Examining of adhesive properties between COP and EPDM in the three-dimensional microchemical chip)

### (1) Producing of a three-dimensional microchemical chip by joining of COP (cycloolefin polymer) and EPDM sheet

A microchemical chip 1 shown in Fig. 6 was produced by employing cycloolefin polymer plates 10, 30 as flow-path-retaining substrate sheets and an EPDM sheet 20 as a flow-path-supporting substrate sheet. The cycloolefin polymer plates 10, 30 were made from ZEONOR (registered trademark) 1060R (manufactured by Zeon Corporation) and had a size of 30×40 mm and 2 mm thickness. The EPDM sheet 20 had the size of the same as the cycloolefin polymer plates 10, 30 and 500 µm thickness. The EPDM sheet 20 was formed by the composition of the same as Example 2 employing EPT3072 (manufactured by Mitsui Chemicals, Inc., trade name). A flow path 22 was defined on the EPDM sheet 20 by using a laser processing apparatus (supplied by COMNET Corporation, trade name: LaserPro SPILIT, conditions for processing: speed 6.0, power 90, PPI 400) as shown in Fig. 6. The flow path 22 included fluid-sample-injecting sites 21 a, 21 b and fluid-sample-draining sites 22a, 22b, 22c having each 1 mm diameter. The flow path 22 had a branching channel shape having 500 µm width. The formed EPDM sheet 20 was washed with ethanol. A surface of the formed EPDM sheet 20 was activated by three times of the corona discharge treatment of which conditions were 1 mm gap length, 13.5 kV voltage and 70 mm/second. Thereafter, the EPDM sheet 20 was dipped into 1.0% by weight of 3-mercaptopropyltrimethoxysilane of the silane-coupling agent in the ethanol solution, dried by using a blow-gun, heated at 80°C for 10 minutes and washed with ethanol again. Fluid-sample-injecting holes 11 a, 11b and fluid-sample-draining holes 12a, 12b, 12c were drilled into the cycloolefin polymer plate 10 for covering. The cycloolefin polymer plate 10 for covering and the cycloolefin polymer plate 30 for bottom face supporting were washed with ethanol. Thereafter, the cycloolefin polymer plates 10, 30 were dipped into 0.1% by weight of 2,6-diazide-4-{3-(triethoxysilyl)propylamino}-1,3,5-triazine (P-TES) in an ethanol solution, dried by using the blow-gun and irradiated with UV ray (200 mJ/cm²: 254 nm). The EPDM sheet 20 was sandwiched between the cycloolefin polymer plates 10, 30 so that the fluid-sample-injecting sites 21 a, 21b and the fluid-sample-draining sites 22a, 22b, 22c were corresponded to the fluid-sample-injecting holes 11a, 11b and the fluid-sample-draining holes 12a, 12b, 12c, respectively. The resultant products were pressed by thermal compression at 80°C for 10 minutes while pressing 70 kgf. The microchemical chip 1 was obtained.

### (2) Pressure resistance test

The fluid-sample-injecting hole 11b and the fluid-sample-draining holes 12a, 12b, 12c were closed, and pressurized air was introduced into the fluid-sample-injecting site 21 a through the fluid-sample-injecting hole 11a. In the result, the microchemical chip 1 exhibited pressure resistance up to 1.5 MPa.

### (Example 6: Examining of fluorescence intensity)

### (1) Preparing of a reflectivity-untreated silicone rubber sheet

100 parts by weight of a methylvinylsilicone rubber as silicone rubber (manufactured by Dow Corning Toray Co., Ltd., trade name: SH851U) and 0.5 parts by weight of 2,5-dimethyl-2,5-di(tert-butylperoxy)hexane as a peroxide type crosslinking agent (manufactured by Dow Corning Toray Co., Ltd., trade name: PC-4, 50% silica solution) were kneaded. A composite for a sheet was obtained. The composite was heated and pressurized, and a silicone-made sheet was prepared.

### (2) Preparing of high reflectivity silicone rubber

100 parts by weight of methylvinylsilicone rubber as silicone rubber (manufactured by Dow Corning Toray Co., Ltd., trade name: SH851U) and 0.5 parts by weight of 2,5-dimethyl-2,5-di(tert-butylperoxy)hexane as a peroxide type crosslinking agent (manufactured by Dow Corning Toray Co., Ltd., trade name: PC-4, 50% silica solution) and 50 part by weight of rutile-type titanium oxide (manufactured by ISHIHARA SANGYO KAISHA, LTD., trade name: CR-58) were kneaded. A composite for a sheet was obtained. The composite was heated and pressurized, and a high reflectivity silicone rubber sheet was prepared.

### (3) Producing of a three-dimensional microchemical chip by joining of the silicone rubber sheet or the high reflectivity silicone rubber sheet and the COP sheet

A microchemical chip shown in Fig. 5 was produced by employing a cycloolefin polymer (COP) sheet 10 (manufactured by Zeon Corporation, trade name: ZEONOR (registered trademark) film ZF16-100) as flow-path-retaining substrate sheet for upper face side, the high reflectivity silicone rubber sheets 20, 30 having 1.0 mm thickness as a flow-path-supporting substrate sheet and flow-path-retaining substrate sheet for bottom face side. A flow path 22 having 1 mm width was defined into the high reflectivity silicone rubber sheet by using a laser processing apparatus (supplied by COMNET Corporation, trade name: LaserPro SPILIT, conditions for processing: speed 6.0, power 90, PPI 400). The flow path 22 included a fluid-reagent-receiving site 21a and fluid-reagent-draining site 21b having each 1 mm diameter and fluid accumulation part having 5 mm diameter which was expanded midway part of the flow path 22 as shown in Fig. 1. The formed high reflectivity silicone rubber sheet was washed with ethanol. A surface of the high reflectivity silicone rubber sheet was activated by three times of the corona discharge treatment of which conditions were 1 mm gap length, 13.5 kV voltage and 70 mm/second. A fluid-sample-injecting hole 11a and fluid-sample-draining hole 12a were opened into the cycloolefin polymer sheet 10 for covering by using the laser processing apparatus in the same as above (conditions for processing: speed 10, power 15, PPI 400). The processed substrate sheet 10 for covering was washed with ethanol. A surface of the substrate sheet was activated by three times of a corona discharge treatment of which conditions were 1 mm gap length, 13.5 kV voltage and 70 mm/second. The substrate sheet 10 was dipped into the 3-(2-aminoethylamino)propyltrimethoxysilane of a silane-coupling agent in the ethanol solution, dried by using a blow-gun, heated at 80°C for 10 minutes and washed with ethanol again. The high reflectivity silicone rubber sheet 30 for bottom face supporting was washed with ethanol. A surface of the cycloolefin polymer sheet 30 was activated by three times of the corona discharge treatment in the same manner as above. The high reflectivity silicone rubber sheet 20 was sandwiched between the substrate sheets 10, 30 so that the fluid-sample-injecting site 21 a and the fluid-sample-draining site 21b were corresponded to the fluid-sample-injecting hole 11 and the fluid-sample-draining hole 13, respectively. The resultant products were pressed by thermal compression at 80°C for 10 minutes while pressing 70 kgf. In the result, the microchemical chip 1 was obtained.

### (4) Measuring of fluorescence

A microchemical chip for comparative example was produced in the same manner as above except that the reflectivity-untreated silicone rubber sheet, which was prepared by employing SH851U, instead of the high reflectivity silicone rubber. Fluorescence intensities of the microchemical chip 1 having the high reflectivity silicone rubber sheet produced as above and the microchemical chip for comparative example were measured, and obtained results were compared. 35 µL of 0.01% by weight of a fluorescence colorant LUMOGEN F ORANGE 240 in the ethanol solution was injected into the fluid-reagent-injecting hole 11a. The fluid-reagent-injecting hole 11a and the fluid-reagent-draining hole 12a were closed with a tape. A ray of 500 nm in main wavelength was irradiated to the fluid accumulation part of the microchemical chip 1 into which the fluorescence colorant was injected. The fluorescence intensity was measured by using a flash multi photometer system (manufactured by Otsuka Electronics Co., Ltd., model: MCPD-7000). The fluorescence intensity of the microchemical chip 1 having the high reflectivity silicone rubber sheet was quantified relative to 100 of the fluorescence intensity of the microchemical chip having the reflectivity-untreated silicone rubber sheet. The result is shown in Table 3.

**Table 3 Comparison of fluorescence intensity**

| | Microchemical chip having Reflectivity-untreated silicone rubber sheet | Microchemical chip having High reflectivity silicone rubber sheet |
|---|---|---|
| Relative quantity (Peak intensity) | 100 | 629 |

As shown in Table 3, in the peak intensity, it is obvious that the fluorescence intensity of the microchemical chip 1 having the high reflectivity silicone rubber sheet is improved 6.29 times relative to the fluorescence intensity of the microchemical chip having the reflectivity-untreated silicone rubber sheet.

### (Example 7: Examining of thermal conductivity and thermal insulation)

### (1) Preparing of silicone rubber sheet

100 parts by weight of a methylvinylsilicone rubber as silicone rubber (manufactured by Dow Corning Toray Co., Ltd., trade name: SH851U) and 0.5 parts by weight of 2,5-dimethyl-2,5-di(tert-butylperoxy)hexane as a peroxide type crosslinking agent (manufactured by Dow Corning Toray Co., Ltd., trade name: PC-4, 50% silica solution) were kneaded. A composite for a sheet was obtained. The composite was heated and pressurized, and a silicone rubber sheet was prepared.

### (2) Preparing of thermally conductive silicone rubber sheet

50 parts by weight of methylvinylsilicone rubber as a silicone rubber (manufactured b) Dow Corning Toray Co., Ltd., trade name: SH1005), 50 parts by weight of polydimethylsiloxane as silicone oil (manufactured by Dow Corning Toray Co., Ltd., trade name: SH200 100cs), 50 parts by weight of magnesium oxide as thermally conductive filler (manufactured by Kyowa Chemical Industry Co., Ltd., trade name: PYROKISUMA (registered trademark) 5301, average particle size: 2 µm), 200 parts by weight of magnesium oxide (manufactured by Kyowa Chemical Industry Co., Ltd., trade name: PYROKISUMA (registered trademark) 3320, average particle size: 20µm), 50 parts by weight of aluminum hydroxide Al(OH)₃ as an additive agent (manufactured by Showa Denko K.K., trade name: HIGILITE (registered trademark) H32), 10 parts by weight of calcium oxide CaO (manufactured by Inoue Calcium Corporation, trade name: VESTA PP) and 0.01 parts by weight of a platinum carbonyl cyclovinylmethylsiloxane complex in a vinylmethylcyclosiloxane solution (manufactured by Gelest, Inc.) of a platinum complex as a platinum catalyst were kneaded. A composite for a silicone rubber-made thermally conductive sheet was obtained. The composite was heated and pressurized, and the silicone rubber-made thermally conductive sheet for flow-path-retaining substrate sheet was produced.

### (3) Preparing of foamable silicone rubber sheet

Foamable silicone rubber was prepared according to Example 1 in Japanese Application Publication No. 2008-94981. 2 parts by mass of a peroxide type vulcanizing agent was added into 100 parts by mass of the silicone rubber, followed by mixing and dispersing by using a biaxial roller. A silicone rubber composite thereby was prepared. 10 parts by mass of trimethylolpropane as a first pore-forming agent and 390 parts by mass of pentaerythritol as a second pore-forming agent were added into 110 parts by weight of the silicone rubber composite containing a vulcanizing agent. The resultant mixture was kneaded for 10 minutes by using a kneader maintained at a kneading temperature (T1) of 110°C and thus, the pore-forming agents were mixed and dispersed. A rubber composite was obtained. By compressing the rubber composite, which was obtained through kneading process, for 10 minutes in a mold for press processing maintained at a vulcanizing temperature (T2) of 170°C, a sheet like vulcanized rubber composite having 2 mm thickness was obtained. The difference (T1-T2) between the vulcanizing temperature (T2) and the kneading temperature (T1) was -60°C. The sheet like vulcanized rubber composite was washed with hot water, and the pore-forming agents were dissolved from the vulcanized rubber. The foamable silicone rubber as porous material was obtained.

### (4) Measuring of thermal insulation

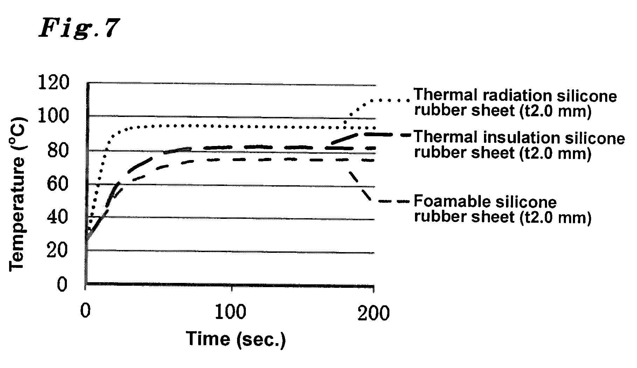

The silicone rubber sheet, the thermal conductive silicone rubber sheet and the foamable silicone rubber sheet having each 2.0 mm thickness (t) were put on a metal plate heated at 100°C, and rising temperature of time-dependence was measured. The results are shown in Fig. 7.

As shown in Fig. 7, the temperature of the thermally conductive silicone rubber sheet is risen for a short time, and the temperature of the foamable silicone rubber sheet is slowly risen. When the exoergic rubber material is employed in the three-dimensional microchemical chip, the three-dimensional microchemical chip can correspond immediately to temperature change. When conducting a thermal cycle, reduction of time therefore is achieved. In the case of setting various temperatures in the three-dimensional microchemical ship, when the foamable rubber material is employed therein, interference from temperature can be decreased.

### Industrial Applicability

The three-dimensional micro chemical chip of the present invention has superior thermal radiation properties from inside thereof and superior thermal conductivity properties from outside thereof. The three-dimensional micro chemical chip can be used in an analysis of biological component of patients at an emergency medical practice which requires to obtain a result of the analysis rapidly; a DNA analysis for identification of DNA using a electrophoresis after extracting DNA from things left behind such as a trace amount of a bloodstain, biological fluid, hair and a biological tissue cell etc. at a crime scene, and conducting a PCR amplification for amplifying DNA; an evaluation of physical properties and drug efficacy of various drug candidates for searching a novel drug; diagnosis for custom-made medical treatment; microsynthesis of peptide, DNA or a functional low-molecular, culturing and amplifying of stem cells and virus.

The three-dimensional micro chemical chip can be used in custom-made medical care, identification by a DNA analysis of various flora and fauna etc., because the flow path can be easily defined so as to have various shapes.

The obtained microchemical chip by the method for producing the three-dimensional microchemical chip of the present invention after installing it onto a microreactor or analysis apparatus can be used to conduct a genetic diagnosis or healing in a medical field; various analyses in a criminal investigation field by using biological reagent; microbiological search by using an underwater apparatus in a remote location such as the ocean or lake and a reservoir etc.; and various syntheses of drug development.

### Explanations of Letters or Numerals

Numerals mean as follows. 1: three-dimensional microchemical chip, 10: flow-path-retaining substrate sheet, 11, 11 a, 11b, 11e, 11g: fluid-sample-injecting hole, 13, 13a, 13b: fluid-sample-draining hole, 14a: first face, 14b: second face, 20: flow-path-supporting substrate sheet, 21, 21 a, 21b, 21c, 21d, 21 e, 21 g, 21i: fluid-sample-receiving hole, 22, 22a, 22b, 22d, 22e, 22f, 22g, 22i: micro flow path, 22d': fluid accumulation part, 23, 23a, 23b: fluid-sample-delivering hole, 24a: first face, 24b: second face, 30: flow-path-retaining substrate sheet, 31, 31 c, 31 d, 31 h, 31 i: fluid-sample-receiving hole, 34a: first face, 34b: second face, 40: flow-path-supporting substrate sheet, 41, 41c, 41d, 41h, 41i: fluid-sample-receiving hole, 42, 42a, 42C, 42h, 41h': micro flow path, 44a: first face, 44b: second face, 50: flow-path-retaining substrate sheet, 54a: first face, 60a, 60b: holder, 61 a, 61 b, 61e, 61g: injection leading hole, 63a, 63b: drain leading hole, A: injecting side, B: draining side

## Claims

1. A three-dimensional microchemical chip comprising:
a single or plural flow-path-supporting substrate sheet made from rubber, resin, metal, ceramics and/or glass;
a flow-path-retaining substrate sheet made from rubber, resin, metal, ceramics and/or glass, and retains to contact and stack the flow-path-supporting substrate sheet at most upper face and/or most lower face thereof;
a surface of at least one of the flow-path-supporting substrate sheet and the flow-path-retaining substrate sheet which joins and integrates these sheets by a direct covalent bond and/or indirect covalent bond interposing a molecular adhesive via a treatment at least one of a dry treatment selected from the group consisting of a corona treatment, plasma treatment and ultraviolet irradiation treatment, and a molecular adhesive treatment;
a flow path, defined by recessing and/or piercing the flow-path-supporting substrate sheet, in which a fluid sample, selected from the group consisting of a specimen, reagent and sample, is subjected to a chemical reaction and/or chemical action by flowing the fluid sample thereinto through pressurization and/or capillarity phenomenon thereof; and
a receiving hole which is pierced in the flow-path-retaining substrate sheet covering the flow path and is connected to the flow path,
the flow path and the receiving hole are sequentially and sterically connected from a fluid-sample-injecting hole to a fluid-sample-draining hole.

2. The three-dimensional microchemical chip according to claim 1, wherein at least any one of the flow path of the plural flow-path-supporting substrate sheets is folded, bent and/or curved at least one location of a midway part thereof.

3. The three-dimensional microchemical chip according to claim 1, wherein diatomaceous earth, mica, talc and/or kaolin is included in at least any one of the flow-path-supporting substrate sheet and the flow-path-retaining substrate sheet.

4. The three-dimensional microchemical chip according to claim 1, wherein the flow-path-supporting substrate sheet and the flow-path-retaining substrate sheet are plurally and alternately stacked each other.

5. The three-dimensional microchemical chip according to claim 1, wherein any one of the flow path of the plural flow-path-supporting substrate sheets is folded back from a draining side toward an injecting side at least one location of a midway part thereof.

6. The three-dimensional microchemical chip according to claim 1, wherein the flow path on the plural flow-path-supporting substrate sheets is parallel arranged, diagonally crossed and/or not diagonally crossed in different level in at least one part thereof each other.

7. The three-dimensional microchemical chip according to claim 1, wherein any one of the flow-path-supporting substrate sheet and the flow-path-retaining substrate sheet is a silicone rubber-made thermal radiation sheet including thermally conductive filler powder of at least one selected from the group consisting of aluminum oxide, magnesium oxide, zinc oxide, graphite carbon, silicon nitride, boron nitride and aluminum nitride.

8. The three-dimensional microchemical chip according to claim 1, wherein the molecular adhesive is included in any one of the flow-path-supporting substrate sheet and the flow-path-retaining substrate sheet.

9. The three-dimensional microchemical chip according to claim 1, wherein the flow-path-supporting substrate sheet and the flow-path-retaining substrate sheet are joined through the molecular adhesive on the surface of these sheets.

10. The three-dimensional microchemical chip according to claim 1, wherein the molecular adhesive contains a silane coupling agent having 6 to 12 carbon atoms and a vinylmethoxysilyl group.

11. The three-dimensional microchemical chip according to claim 1, wherein a flame retardant of at least one selected from the group consisting of antimony trioxide and aluminum hydroxide is contained in any one of the flow-path-supporting substrate sheet and the flow-path-retaining substrate sheet.

12. The three-dimensional microchemical chip according to claim 1, wherein the covalent bond is an ether bond.

13. The three-dimensional microchemical chip according to claim 1, wherein at least one part of the flow-path-supporting substrate sheet or the flow-path-retaining substrate sheet is a foamable silicone rubber sheet made from at least any one from the group consisting of a silicone rubber raw material composite including glass beads and/or zeolite and a silicone composite including a water soluble alcohol.

14. The three-dimensional microchemical chip according to claim 1, wherein an) one of the flow-path-supporting substrate sheet and the flow-path-retaining substrate sheet is a high-reflective silicone rubber sheet, having 80 to 100% of reflectivity, made from silicone rubber into which anatase-type or rutile-type titanium oxide is dispersed.

15. The three-dimensional microchemical chip according to claim 1, wherein any one of the flow-path-supporting substrate sheet and the flow-path-retaining substrate sheet is a low-gas-permeable silicone rubber sheet, having 500 to 0.05 (cc/cm²/mm/sec/cm·Hg×10¹⁰) of gas permeability of at least any gas of oxide gas, nitrogen gas, carbon dioxide gas and water vapor, which is made from silicone rubber, ethylene-propylene-diene-methylene copolymer rubber, butyl rubber, acrylonitrile-butadiene copolymer rubber, fluoro-rubber, styrene-butadiene copolymer rubber and/or hydrin rubber.

16. The three-dimensional microchemical chip according to claim 3, wherein at least any one of the diatomaceous earth, the mica, the talc and the kaolin is scaly-shaped filler.

17. The three-dimensional microchemical chip according to claim 1, wherein at least any one of the flow-path-supporting substrate sheet and the flow-path-retaining substrate sheet is formed by any one selected from the group consisting of peroxide crosslinking silicone rubber, addition crosslinking silicone rubber, condensation crosslinking silicone rubber and radiation crosslinking or electron beam crosslinking silicone rubber, and blended rubber of any one the silicone rubbers and olefin type rubber.

18. The three-dimensional microchemical chip according to claim 1, wherein a part of the flow path is a fluid accumulation part defined by expansion thereof.

19. A method for producing a three-dimensional microchemical chip comprising:
a flow path defining step for defining a flow path for a chemical reaction and/or chemical action, into which a fluid sample selected from the group consisting of a specimen, reagent and sample is flowed by pressurization and/or capillarity phenomenon, in a single or plural flow-path-supporting substrate sheet so as to sequentially and sterically connect from a fluid-sample-injecting hole to a fluid-sample-draining hole;
a receiving hole forming step for producing a flow-path-retaining substrate sheet which is made from rubber, resin, metal, ceramics and/or glass, has a receiving hole connecting to the flow path, and sandwiches the flow-path-supporting substrate sheet;
a treating step for conducting a corona treatment, plasma treatment or ultraviolet irradiation treatment to at least any one of the flow-path-supporting substrate sheet and the flow-path-retaining substrate sheet;
a joining step for stacking the flow-path-supporting substrate sheet and the flow-path-retaining substrate sheet under conditions of normal atmospheric pressure, pressurization or reduced pressure, joining and integrating these sheets via a direct covalent bond and/or indirect covalent bond interposing a molecular adhesive, whereby each of the flow path of the flow-path-supporting substrate sheet is sequentially and sterically connected from the fluid-sample-injecting hole the fluid-sample-draining hole.

20. The method for producing the three-dimensional microchemical chip according to claim 19 comprising:
a step for forming the flow-path-supporting substrate sheet so that at least one location in at least any one of the flow path of the flow-path-supporting substrate sheet is folded, bent and/or curved halfway.
